# EUROPEAN PATENT APPLICATION

(11) **EP 4 851 187 A1**
(43) Date of publication of application: **09.12.2026**
(21) Application number: 25749157.1
(22) Date of filing: 31.01.2025
(51) Int. Cl.: C07K 14/755, A61K 35/76, A61K 38/37, A61K 47/46, A61K 48/00, A61P 7/04, C12N 5/10, C12N 15/12, C12N 15/63, C12N 15/864, C12P 21/02

(54) **HIGH SPECIFIC ACTIVITY/HIGH SECRETION EXPRESSION-TYPE FACTOR VIII VARIANT, NUCLEIC ACID ENCODING HIGH EXPRESSION-TYPE FACTOR VIII, AND USE THEREOF**

(30) Priority: 02.02.2024 JP 2024015293
(71) Applicant: Jichi Medical University, Tokyo 102-0093 (JP)
(72) Inventor: OHMORI, Tsukasa, Shimotsuke-shi, Tochigi 329-0498 (JP); KASHIWAKURA, Yuji, Shimotsuke-shi, Tochigi 329-0498 (JP)
(74) Representative: Weickmann & Weickmann PartmbB
(86) International application number: PCT/JP2025/003309
(87) International publication number: WO 2025/164792

(57) **Abstract**

The present invention provides a human factor VIII variant in which at least one amino acid residue selected from the group consisting of K213, S367, and F2196 of wild-type human factor VIII is substituted with other amino acid, and which has high specific activity and/or secretory expression efficiency as compared with the wild-type, a nucleic acid encoding the same, and a therapeutic agent for hemophilia A by using these.

## Description

### [Technical Field]

The present invention relates to a high specific activity/high secretory expression-type human factor VIII variant and a nucleic acid encoding the same, a human factor VIII-encoding nucleic acid of a high expression-type, and uses thereof. More specifically, the present invention relates to a human factor VIII variant with enhanced secretory expression efficiency and/or specific activity compared to the wild type, and a nucleic acid encoding the same, a nucleic acid encoding human factor VIII (including variants) and having expression efficiency enhanced by modification of a nucleotide sequence, and the treatment of hemophilia A using these, and the like.

### [Background Art]

Hemophilia is a disease caused by genetic deficiency of a blood coagulation factor involved in hemostasis. According to the World Federation of Hemophilia (WFH), there are about 400,000 hemophilia patients in the world, and about 6,000 patients exist in Japan. Hemophilia includes hemophilia A caused by deficiency of factor VIII and hemophilia B caused by deficiency of factor IX, and patients with hemophilia A are more numerous at a ratio of 5:1.

Conventionally, hemophilia A has been treated by replacement therapy of factor VIII. However, the half-life of the coagulation factor is short, and intravenous administration 2 to 3 times per week from childhood is required for prevention of bleeding. In addition, when factor VIII is continuously administered to a hemophilia A patient, an antibody against factor VIII called an inhibitor may arise in the patient (25 to 30%), and it is known that the efficacy of replacement therapy is impaired thereby.

In recent years, as a therapeutic agent for hemophilia A, an antibody drug emicizumab, which acts as a substitute for factor VIII, has been launched on the market. Emicizumab can treat hemophilia A by subcutaneous injection at a lower frequency, once per month, than a factor VIII preparation. However, it is not different from conventional coagulation factor preparations in that permanent administration is necessary., In addition, due to recombinant production by animal cells, the manufacturing cost is high and the burden on the medical economy is large.

Therefore, gene therapy using a viral vector, by which coagulation factor activity is expected to be maintained over a long period by single administration, has attracted attention as a new treatment method for hemophilia. Hemophilia is a hereditary disease caused by a single gene deficiency, and has long been considered to be a good target disease for gene therapy, for reasons that the therapeutic effect can be easily confirmed by the coagulation factor level in blood and that an effect can be expected even by a relatively slight increase in the blood level. In addition, it has also been shown in preclinical studies that continuous expression of a coagulation factor by gene therapy induces immune tolerance mediated by regulatory T cells, and inhibitors can be removed. Recently, partially successful examples of human gene therapy using an adeno-associated virus (AAV) vector have been reported one after another (see, for example, Non Patent Literatures 1 and 2). Although coagulation factors are produced in the liver, an AAV vector can deliver a gene to the liver with high efficiency by intravenous administration.

In the clinical trial by BioMarin, high factor VIII activity was maintained even after one year in the high-dose administration group (Non Patent Literature 1), but after three years, a tendency toward decrease in the blood level was observed. As one of the causes thereof, it has been pointed out that factor VIII expression from a high-dose AAV vector in liver cells may induce endoplasmic reticulum stress (Non Patent Literature 3).

On the other hand, in the clinical trial by Spark, an AAV vector at a lower dose than that of BioMarin was administered using a serotype having high liver tropism, but in the 2×10¹² vg/kg administration group, hepatotoxicity was observed (Non Patent Literature 2). Attempts have been made to reduce the dose and enhance safety by realizing high expression through use of a liver-specific promoter and codon optimization of factor VIII. However, when high expression of the protein itself impairs long-term stability of the therapeutic effect, that approach is not sufficient to deal with the case.

As one means for avoiding attenuation of the therapeutic effect due to high-dose viral vector administration, it is desired to develop a factor VIII variant having high specific activity, which can exhibit coagulation activity equal to or higher than that in the case where wild-type factor VIII is highly expressed at an appropriate protein level, and a factor VIII variant having improved secretion efficiency to the outside of cells, in order to eliminate adverse effects on cells such as endoplasmic reticulum stress. Xiao et al. report that, when 10 amino acid residues in the A1 domain are substituted with porcine types, secretion efficiency is remarkably increased, and among them, 5 amino acid substitutions are important (Patent Literature 1 and Non Patent Literature 4). Furthermore, the same group reports that, when 12 amino acid residues in the human factor VIII light chain are substituted with canine types, specific activity can be increased while maintaining the secretion efficiency of the wild type (Non Patent Literature 5). In addition, Sabatino et al. disclose that, when a furin recognition site in the human factor VIII light chain and one or more amino acids of the a3 domain are substituted with canine types or deleted, specific activity can be increased (Patent Literature 2).

### [Citation List]

### [Patent Literature]

[Patent Literature 1]
   WO 2014/209942
[Patent Literature 2]
   US 2019/0144524

### [Non Patent Literature]

[Non Patent Literature 1]
   Rangarajan, S. et al., N. Eng. J. Med., 377: 2519-2530 (2017)
[Non Patent Literature 2]
   Perrin, G.Q. et al., Blood, 133: 407-414 (2019)
[Non Patent Literature 3]
   Zolotukhin, I. et al., Mol. Ther. Methods Clin. Dev., 3: 16063 (2016)
[Non Patent Literature 4]
   Cao, W. et al., Mol. Ther. Methods Clin. Dev., 19: 486-495 (2020)
[Non Patent Literature 5]
   Firrman, J. et al., Mol. Ther. Methods Clin. Dev., 17: 328-336 (2020)

### [Summary of Invention]

### [Technical Problem]

A first problem of the present invention is to provide a novel human factor VIII variant capable of stably supplementing factor VIII for a long period of time in gene therapy for hemophilia A, and a nucleic acid encoding the same, thereby establishing gene therapy for hemophilia A having more excellent sustained efficacy. A second problem of the present invention is to provide a recombinant factor VIII preparation comprising the variant and an mRNA medicament encoding the variant.

### [Solution to Problem]

The present inventors have conducted intensive studies in an attempt to solve the above-mentioned problems and found that various variants in which at least one amino acid residue selected from the group consisting of lysine at position 213 (K213), serine at position 367 (S367), and phenylalanine at position 2196 (F2196) of wild-type human factor VIII is substituted with another amino acid exhibit high specific activity and/or high secretory expression compared to the wild type (sometimes abbreviated as "high specific activity/high secretory expression-type" in the present specification) (in the present specification, the position of an amino acid residue in human factor VIII is expressed by an amino acid number in a mature full-length single-chain polypeptide (2332 amino acids), and the n-th amino acid residue X (X is a one-letter notation of the amino acid) is abbreviated as "Xn". When n is a negative integer, it indicates the position of an amino acid residue of the signal peptide. In addition, when the n-th amino acid residue X is substituted with another amino acid Y (Y is a one-letter notation of the other amino acid), it is abbreviated as "XnY").

Furthermore, in order to increase the expression efficiency in humans of the variant comprising the above-mentioned amino acid substitutions, the present inventors optimized, for humans, the nucleotide sequence of a nucleic acid encoding the variant using a codon optimization algorithm, but the obtained nucleotide sequence contained a large number of CpG sequences. The present inventors predicted that CpG sequences could induce an immune response in a host and inhibit sustained expression of a transgene, and, without changing the amino acid sequence of the variant, performed nucleotide substitutions that removed these CpG sequences and then performed codon optimization again. The obtained variant-encoding nucleic acid was linked downstream of a promoter sequence from which CpG sequences had likewise been removed, loaded into an AAV vector, and administered to a human liver cell line and hemophilia model mice. As a result, the expression of factor VIII was markedly increased as compared with that from the variant-encoding nucleic acid before removal of the CpG sequences. In order to demonstrate that the expression-enhancing effect resulting from removal of CpG sequences was not limited to the variant but was common to any human factor VIII, including wild-type human factor VIII, the expression of factor VIII was similarly compared between a codon-optimized nucleic acid encoding wild-type human factor VIII and a nucleic acid that had been subjected to removal of CpG sequences and then subjected again to codon optimization. As a result, marked enhancement of expression was likewise observed as a result of the removal of CpG sequences.

The present inventors have conducted further studies based on these findings and completed the present invention.

Accordingly, the present invention provides the following.

### [Item 1]

A human factor VIII variant in which at least one amino acid residue selected from the group consisting of K213, S367, and F2196 of wild-type human factor VIII is substituted with other amino acid, and which has high specific activity and/or secretory expression efficiency as compared with the wild-type.

### [Item 2]

The variant according to item 1, wherein the amino acid substitution at K213 is K213N or K213H, and/or
the amino acid substitution at S367 is S367P, S367N or S367Q, and/or
the amino acid substitution at F2196 is F2196L or F2196M.

### [Item 3]

The variant according to item 2, comprising amino acid substitutions of K213N, S367P, and F2196L.

### [Item 4]

The variant according to any one of items 1 to 3, wherein at least one amino acid residue selected from the group consisting of R-5, P25, A28, L152, M217, W228, Q410, Y487, R489, F501, M539, I566, L603, I642, S727, A736, S1657, Q1659, E1661, I1668, D1681, R1776, H1859, A1993, H2007, N2019, K2085, K2207, F2275, S2296, V2314, Q2316 and M2321 is further substituted with other amino acid.

### [Item 5]

The variant according to item 4, comprising at least one amino acid substitution selected from the group consisting of R-5P, P25H, A28T, L152A, L152D, L152E, L152H, L152I, L152M, L152N, L152P, L152Q or L152V, M217T, W228Q, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, K2207Q, F2275L, S2296A, V2314A, Q2316H, and M2321L.

### [Item 6]

The variant according to item 3, further comprising amino acid substitution of L152A, L152D, L152E, L152H, L152I, L152M, L152N, L152P, L152Q, or L152V, and/or
I1668F and D1681G.

### [Item 7]

The variant according to item 1, wherein the amino acid substitution at K213 is K213N,
the variant further comprises amino acid substitution of M539L, and optionally at least one amino acid substitution within the A2 domain selected from the group consisting of I566M, L603P, and I642V, and
further comprises amino acid substitution of K2207Q, Q2316H, and M2321L, and optionally at least one amino acid substitution within the C2 domain selected from the group consisting of F2275L, S2296A, and V2314A.

### [Item 8]

The variant according to item 7, further comprising amino acid substitutions of I1668F and D1681G.

### [Item 9]

The variant according to item 3, further comprising amino acid substitutions of L152P, Y487H, and L603P, and optionally at least one amino acid substitution selected from the group consisting of S727P, Q1659E, H1859R, A1993V, H2007Q, N2019K, and K2085M.

### [Item 10]

The variant according to item 3, further comprising amino acid substitutions of M539L, I566M, L603P, I642V, K2207Q, F2275L, S2296A, V2314A, Q2316H, and M2321L.

### [Item 11]

The variant according to item 10, further comprising amino acid substitutions of I1668F and D1681G.

### [Item 12]

A human factor VIII variant having the following amino acid substitutions (a) to (n) in wild-type human factor VIII:
(a) K213N, S367P and F2196L
(b) K213N, S367P, F2196L, I1668F and D1681G
(c) K213N, M539L, I566M, I642V, K2207Q, F2275L, Q2316H and M2321L
(d) K213N, M539L, I566M, L603P, I642V, F2196L, K2207Q, F2275L, Q2316H and M2321L
(e) K213N, M539L, I566M, I642V, F2196L, K2207Q, F2275L, S2296A, V2314A, Q2316H and M2321L
(f) K213N, M539L, I566M, I642V, I1668F, D1681G, F2196L, K2207Q, F2275L, S2296A, V2314A, Q2316H and M2321L
(g) L152P, K213N, S367P and F2196L
(h) L152P, K213N, S367P, Y487H, L603P and F2196L
(i) L152P, K213N, S367P, Y487H, L603P, S727P, Q1659E and F2196L
(j) L152P, K213N, S367P, Y487H, L603P, S727P, Q1659E, H1859R, A1993V, H2007Q, K2085M and F2196L
(k) K213N, S367P, M539L, I566M, L603P, I642V, F2196L, K2207Q, F2275L, S2296A, V2314A, Q2316H and M2321L
(l) P25H, A28T, K213N, M217T, S367P, Q410L, Y487H, F501M, M539L, I566M, L603P, I642V, S727P, A736V, S1657P, Q1659E, E1661K, I1668F, D1681G, R1776K, N2019K, F2196L, K2207Q, F2275L, S2296A, V2314A, Q2316H and M2321L
(m) R-5P, P25H, A28T, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, N2019K, F2196L, K2207Q, F2275L, S2296A, V2314A, Q2316H and M2321L
(n) R-5P, P25H, A28T, L152P, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, F2196L, K2207Q, F2275L, S2296A, V2314A, Q2316H and M2321L.

### [Item 13]

The variant according to any one of items 1 to 12, wherein the B domain is deleted.

### [Item 14]

The variant according to item 13, wherein the deletion of the B domain is a deletion of the amino acid sequence from position 763 to position 1656 in the amino acid sequence represented by SEQ ID NO: 1.

### [Item 15]

A nucleic acid encoding the variant according to any one of items 1 to 14.

### [Item 16]

The nucleic acid according to item 15, which is codon-optimized for humans.

### [Item 17]

A nucleic acid that is either the nucleic acid according to item 16 or a nucleic acid encoding wild-type human factor VIII and codon-optimized for humans, and that has been further modified so as not to contain CpG sequences without changing the amino acid sequence encoded thereby.

### [Item 18]

An expression vector comprising the nucleic acid according to any one of items 15 to 17.

### [Item 19]

The expression vector according to item 18, wherein the aforementioned nucleic acid is under the control of a liver-specific promoter.

### [Item 20]

The expression vector according to item 18 or 19, wherein the promoter has been modified so as not to contain CpG sequences.

### [Item 21]

The expression vector according to any one of items 18 to 20, wherein the vector is a viral vector.

### [Item 22]

The expression vector according to item 21, wherein the virus is a liver-tropic adeno-associated virus.

### [Item 23]

A host cell into which the expression vector according to any one of items 18 to 22 has been introduced.

### [Item 24]

A method for producing human factor VIII or a variant thereof, comprising culturing the host cell according to item 23 and recovering human factor VIII or a variant thereof from the obtained culture.

### [Item 25]

A medicament comprising the variant according to any one of items 1 to 14, the nucleic acid according to any one of items 15 to 17, the expression vector according to any one of items 18 to 22, or the host cell according to item 23.

### [Item 26]

The medicament according to item 25, for the treatment of hemophilia A.

### [Item 26a]

A method for treating hemophilia A, comprising administering an effective amount of the variant according to any one of items 1 to 14, the nucleic acid according to any one of items 15 to 17, the expression vector according to any one of items 18 to 22, or the host cell according to item 23 to a patient with hemophilia A.

### [Item 26b]

The variant according to any one of items 1 to 14, the nucleic acid according to any one of items 15 to 17, the expression vector according to any one of items 18 to 22, or the host cell according to item 23, for use in the treatment of hemophilia A.

### [Item 26c]

Use of the variant according to any one of items 1 to 14, the nucleic acid according to any one of items 15 to 17, the expression vector according to any one of items 18 to 22, or the host cell according to item 23, for the manufacture of a therapeutic agent for hemophilia A.

### [Advantageous Effects of Invention]

According to the present invention, a human factor VIII variant having high specific activity and/or high secretory expression efficiency and a nucleic acid encoding the same are provided, and, by using these, treatment of hemophilia A in which factor VIII can be stably supplemented for a long period of time becomes possible. In addition, according to the present invention, a human factor VIII-encoding nucleic acid having enhanced protein expression efficiency as compared with others, even when encoding the same amino acid sequence, is provided.

### [Brief Description of Drawings]

[Fig. 1]

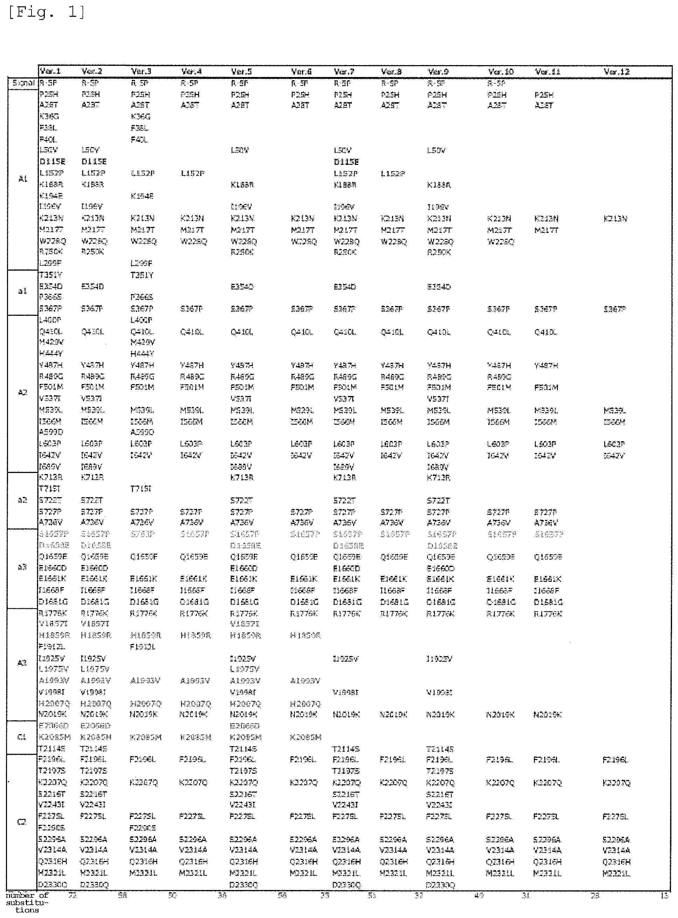

   Fig. 1 shows amino acid substitutions of the variants of the present invention (Ver. 1 to Ver. 12).
[Fig. 2]

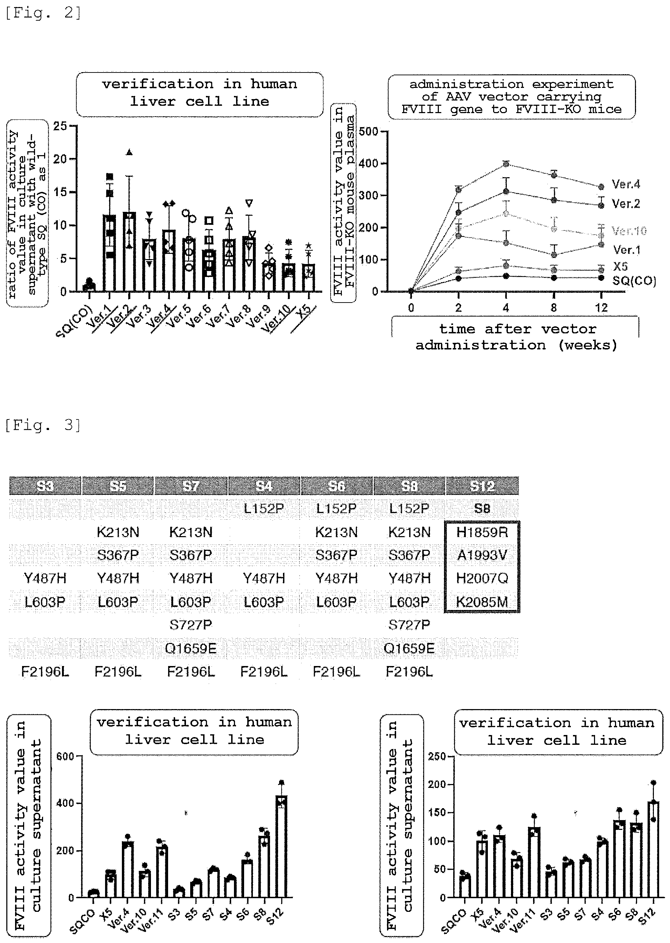

   Fig. 2 shows in vitro (left) and in vivo coagulation factor activities of Ver. 1 to Ver. 10.
[Fig. 3]
   Fig. 3 shows in vitro coagulation factor activities of variants such as S3, S5, S7, S4, S6, S8, and S12 (left: one-stage coagulation method, right: synthetic substrate method).
[Fig. 4]

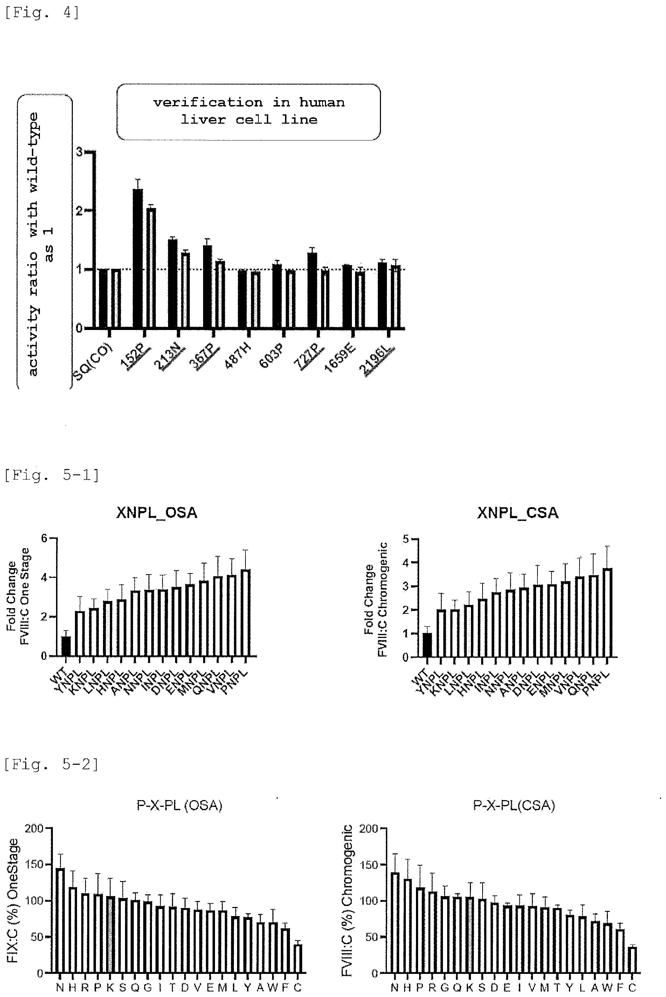

   Fig. 4 shows the effect of each individual amino acid substitution contained in the S8 variant on coagulation factor activity.
[Fig. 5-1]
   Fig. 5-1 shows coagulation factor activities of XNPL variants when L152 was substituted with various other amino acids.
[Fig. 5-2]
   Fig. 5-2 shows coagulation factor activities of PXPL variants when K213 was substituted with various other amino acids.
[Fig. 5-3]

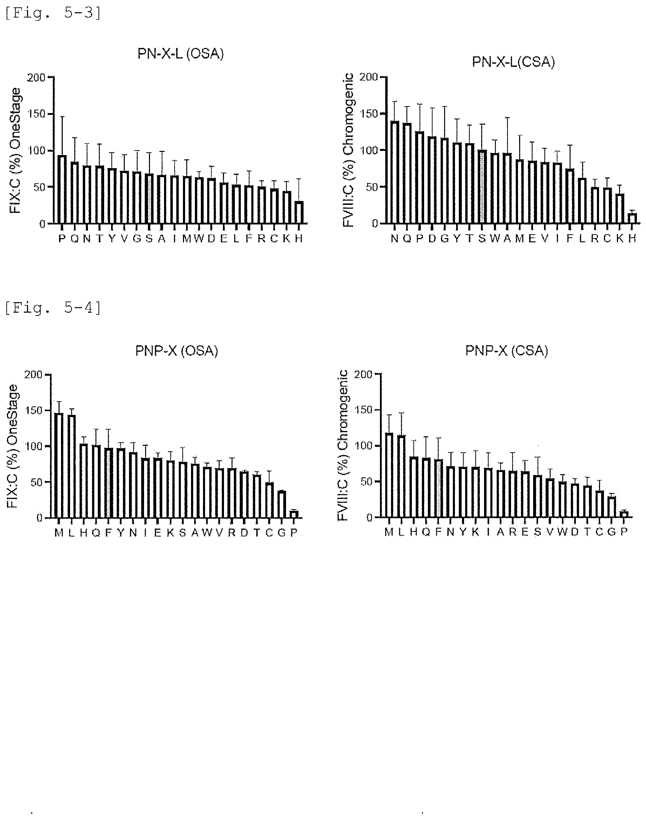

   Fig. 5-3 shows coagulation factor activities of PNXL variants when S367 was substituted with various other amino acids.
[Fig. 5-4]
   Fig. 5-4 shows coagulation factor activities of PNPX variants when F2196 was substituted with various other amino acids.
[Fig. 6]

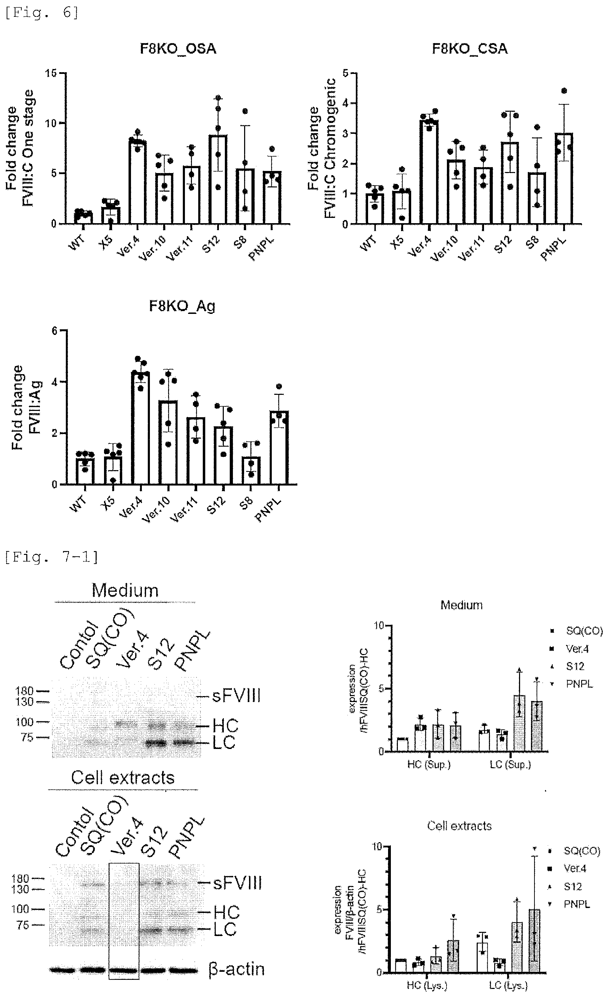

   Fig. 6 shows in vivo coagulation factor activities and blood FVIII antigen amounts of various variants.
[Fig. 7-1]
   Fig. 7-1 shows intracellular and extracellular localization of wild-type and various variant FVIIIs.
[Fig. 7-2]

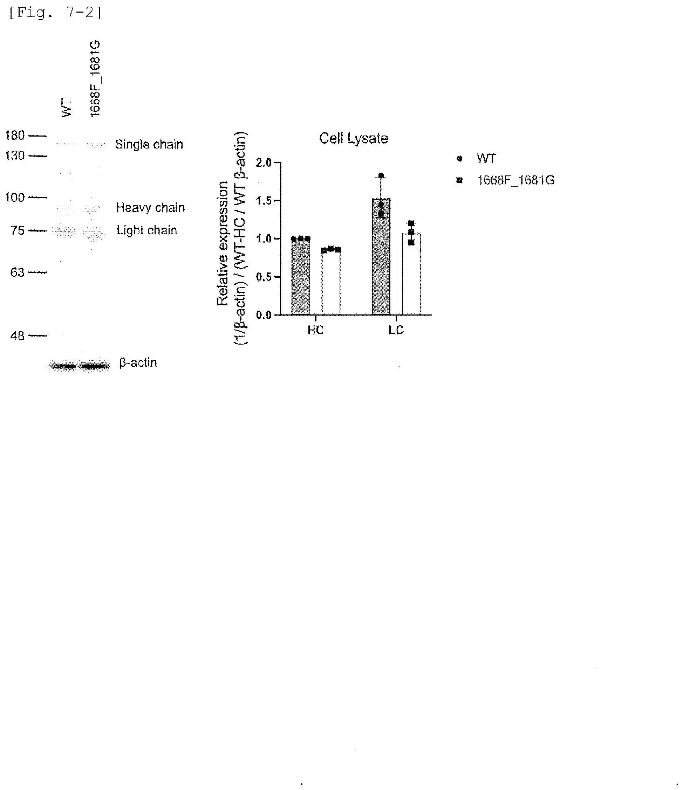

   Fig. 7-2 shows intracellular localization of wild-type and FG mutant FVIIIs.
[Fig. 8]

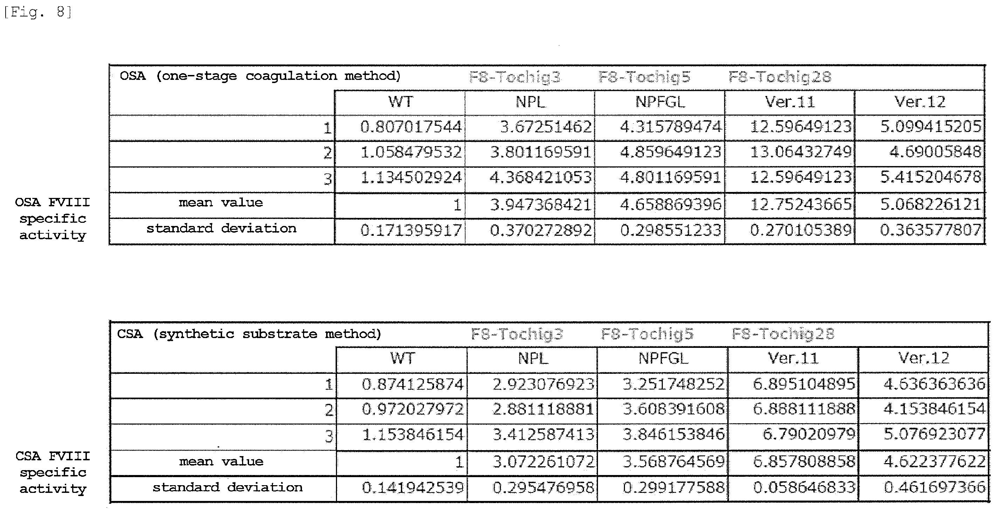

   Fig. 8 shows in vitro coagulation factor activity of variant Ver. 12.
[Fig. 9]

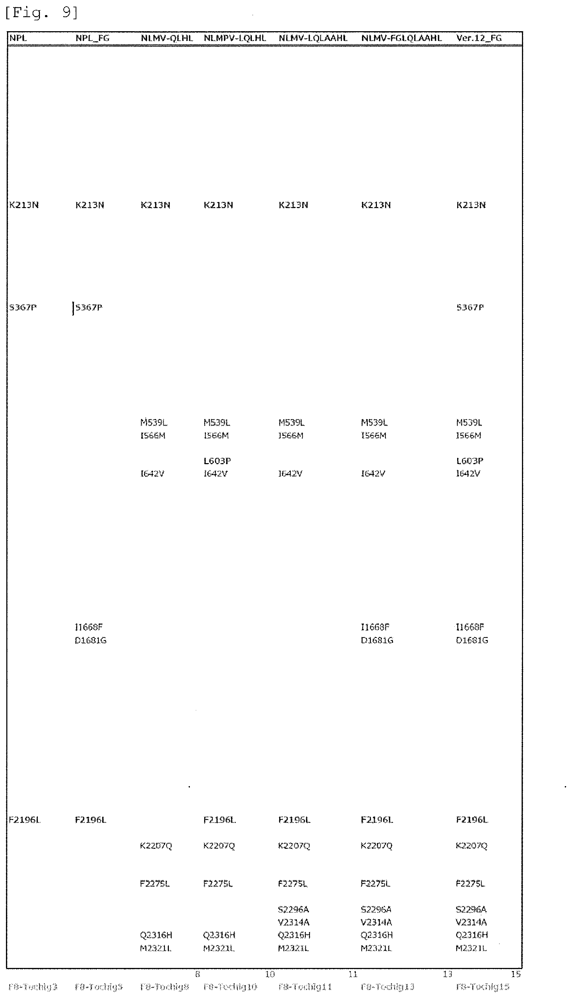

   Fig. 9 shows amino acid substitutions of the variants of the present invention (F8-Tochigi-3, 5, 8, 10, 11, 13, and 15).
[Fig. 10]

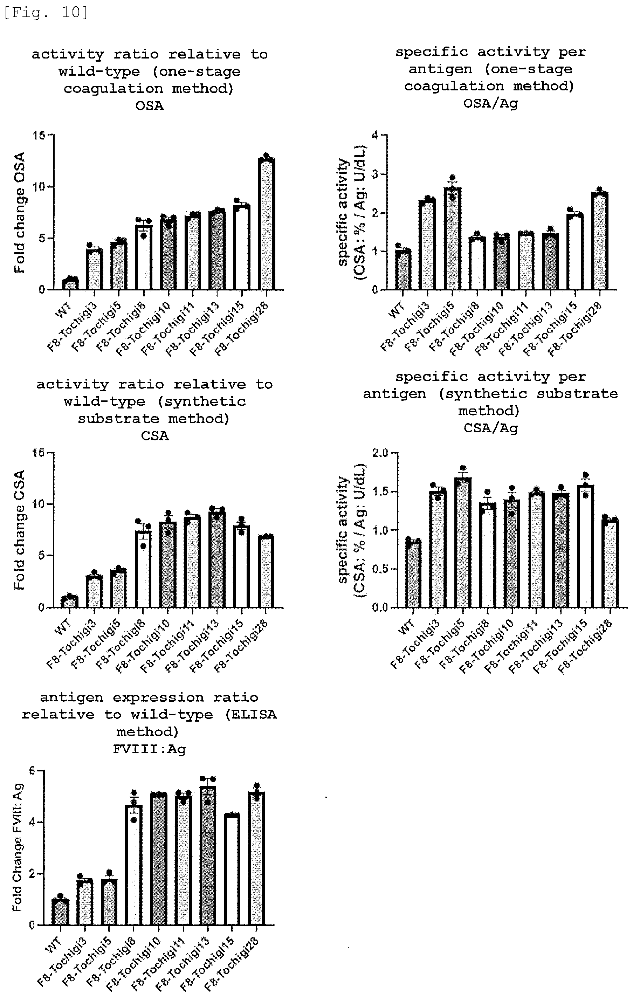

   Fig. 10 shows in vitro coagulation factor activities and antigen amounts of the variants of the present invention (F8-Tochigi-3, 5, 8, 10, 11, 13, 15, and 28).
[Fig. 11-1]

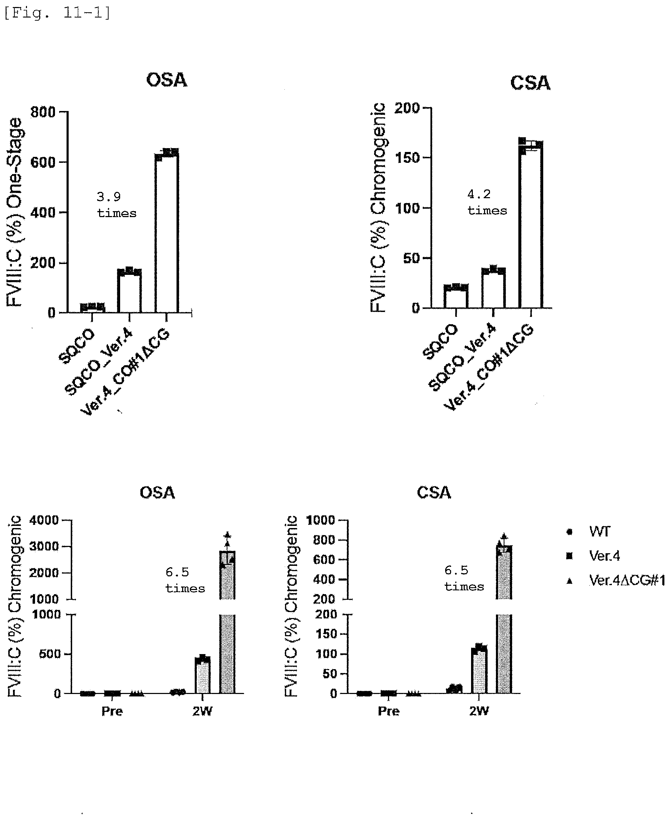

   Fig. 11-1 shows the expression-enhancing effect in vitro and in vivo of the Ver. 4 variant by removal of CpG sequences.
[Fig. 11-2]

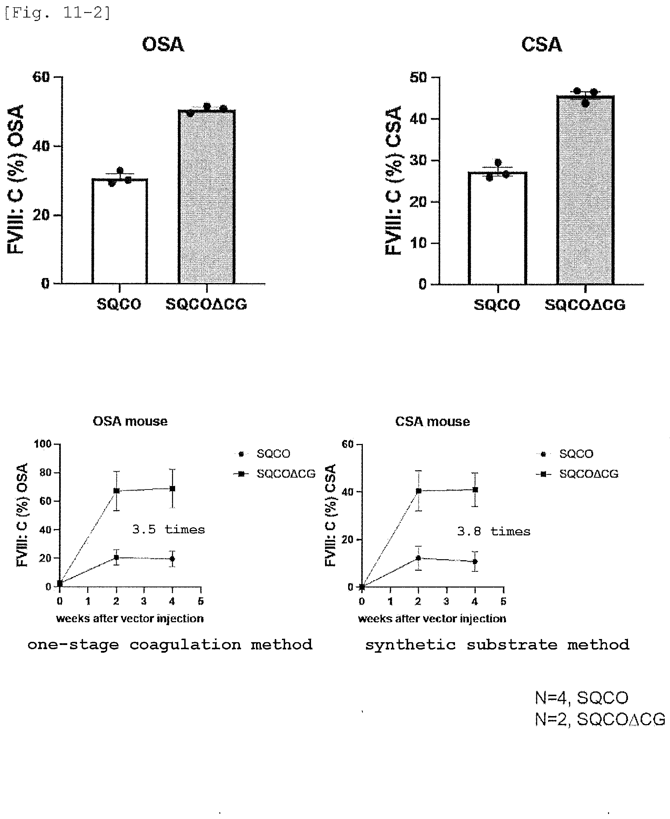

   Fig. 11-2 shows the expression-enhancing effect in vitro and in vivo of wild-type hFVIII by removal of CpG sequences.
[Fig. 12-1]

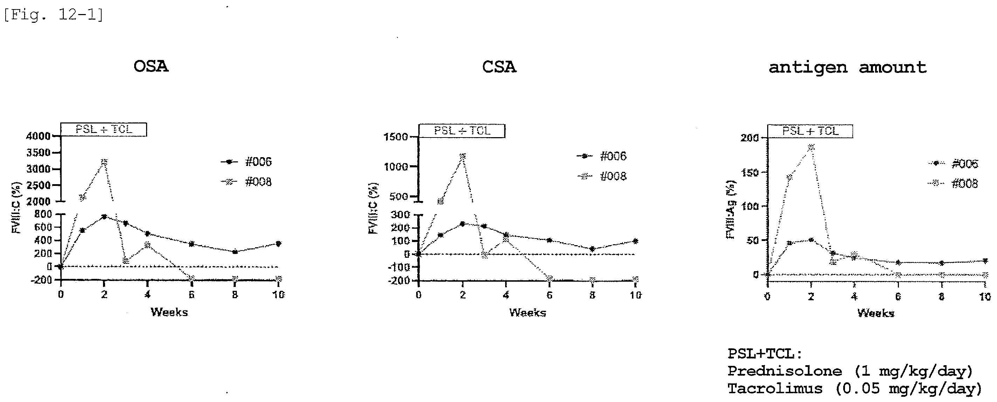

   Fig. 12-1 shows that an AAV8 vector carrying the Ver. 4 variant from which CpG sequences have been removed can markedly increase factor VIII activity in a cynomolgus monkey model at a dose used in a clinical trial.
[Fig. 12-2]

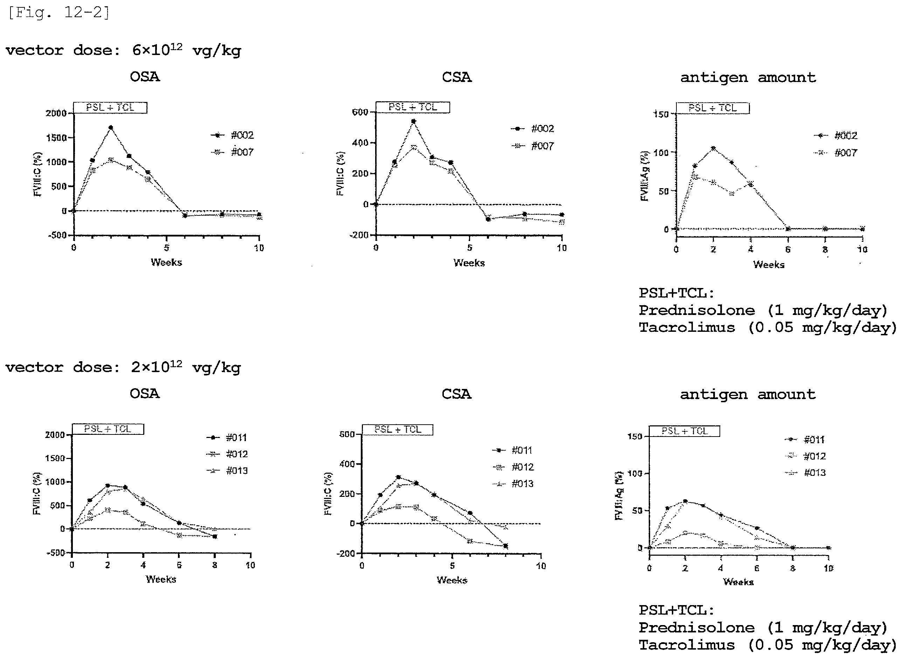

   Fig. 12-2 shows that an AAV5 vector carrying the Ver. 4 variant from which CpG sequences have been removed can markedly increase factor VIII activity in a cynomolgus monkey model at a dose of 1/10 (upper) and a dose of 1/30 (lower) of the dose of a marketed drug.
[Fig. 13]

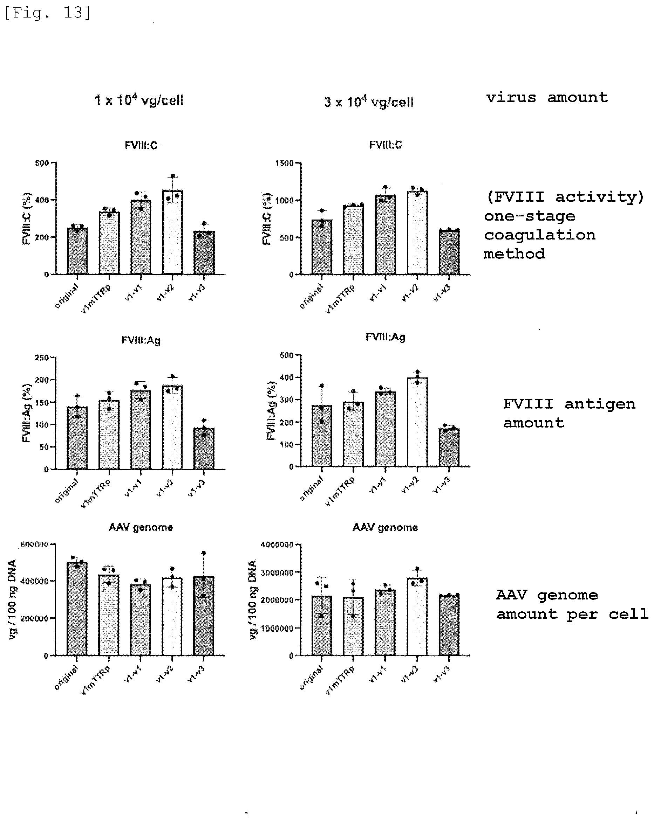

   Fig. 13 shows the factor VIII activity-enhancing effects of truncation of the sequence between a promoter and a human factor VIII-encoding sequence and a polyA addition signal.

### [Description of Embodiments]

A single-chain full-length human factor VIII protein from which a signal peptide consisting of 19 amino acids has been cleaved in the endoplasmic reticulum is composed of three A domains (A1, A2, and A3), two C domains (C1 and C2), B domain, and further three peptide regions rich in acidic amino acids (a1, a2, and a3), and is arranged, from the amino terminus, in the order of A1 (positions 1 to 336), a1 (positions 337 to 372), A2 (positions 373 to 710), a2 (positions 711 to 740), B (positions 741 to 1648), a3 (positions 1649 to 1689), A3 (positions 1690 to 2019), C1 (positions 2020 to 2172), and C2 (positions 2173 to 2332). R1313, R1648, and the like in the B domain are cleaved in the Golgi apparatus, thereby forming two chains, a heavy chain and a light chain. Since coagulation activity is hardly impaired even when the B domain is deleted, B-domain-deleted (BDD) factor VIII is frequently used and is also preferably used in the present invention.

The present invention provides a human factor VIII variant having enhanced specific activity and/or secretory expression efficiency as compared with the wild type (hereinafter also referred to as "the variant of the present invention"). The variant of the present invention is characterized in that at least one amino acid residue selected from the group consisting of K213, S367, and F2196 of wild-type human factor VIII is substituted with another amino acid.

In the present specification, "wild-type human factor VIII" means a protein comprising the amino acid sequence of naturally occurring normal human factor VIII, and specifically includes
(a) a protein consisting of the amino acid sequence represented by SEQ ID NO: 1 (registered in UniProtKB under accession number P00451), or
(b) a protein consisting of the amino acid sequence of an allelic variant (minor allele frequency (MAF) of less than 1%) or a genetic polymorphism (MAF of 1% or more; for example, an SNP or the like) of the protein of (a) (for example, one listed as a variant not associated with a disease in the "Variants" section of UniProtKB accession number P00451 or the like; provided that it has no amino acid mutation at the positions of K213, S367, and F2196), and showing specific activity and secretory expression efficiency equivalent to those of the protein of (a).

Here, the specific activity or secretory expression efficiency being "equivalent" means that the specific activity or secretory expression efficiency is 0.8 to 1.2 times, preferably 0.9 to 1.1 times, and more preferably 0.95 to 1.05 times that of the protein of (a).

In a preferred embodiment, the wild-type human factor VIII is the protein of (a) above.

In the present specification, "human factor VIII variant" means a protein having an amino acid sequence in which one or more amino acids have been substituted, deleted, inserted, or added in the amino acid sequence of the wild-type human factor VIII of (a) or (b) above (reference sequence), and having specific activity and/or secretory expression efficiency substantially changed as compared with human factor VIII consisting of the reference sequence. Here, "substantially changed" preferably means being statistically significantly changed, but also encompasses a case where there is a tendency toward an increase or decrease even without a significant difference.

The variant of the present invention is a human factor VIII variant having high specific activity and/or secretory expression efficiency as compared with the wild type. The specific activity of human factor VIII can be evaluated by measuring coagulation factor activity by a one-stage clotting assay (OSA) or chromogenic substrate assay (CSA) known per se, measuring the protein amount by a protein quantification method known per se (e.g., an immunoassay such as ELISA or the like), and calculating the coagulation factor activity per unit amount of protein. In addition, the secretory expression efficiency of human factor VIII can be evaluated by measuring, by a method known per se (e.g., an immunoassay such as ELISA or the like), the concentration of human factor VIII in a culture supernatant of cells transfected with a nucleic acid encoding the same or in plasma of an animal to which the nucleic acid has been administered.

In the variant of the present invention, at least one, preferably two (e.g., K213 and S367, K213 and F2196, or S367 and F2196), and more preferably three (K213, S367, and F2196), amino acid residues selected from the group consisting of K213, S367, and F2196 of wild-type human factor VIII are substituted with other amino acids. When the wild-type human factor VIII is the protein of the aforementioned (b) and the position of the amino acid residue differs from the position of the relevant amino acid residue in SEQ ID NO: 1 due to deletion or insertion/addition of one or more amino acids, the amino acid residues corresponding to K213, S367, and F2196 can be identified as the amino acid residues respectively corresponding to K213, S367, and F2196 of SEQ ID NO: 1 when the amino acid sequence of (b) and the amino acid sequence of SEQ ID NO: 1 are appropriately aligned using any homology search algorithm.

The other amino acids are not particularly limited as long as the human factor VIII variant obtained as a result of the amino acid substitution has enhanced specific activity and/or secretory expression efficiency as compared with the wild type. As an amino acid substitution at K213, K213N or K213H is preferred, and K213N is more preferred. As an amino acid substitution at S367, S367P, S367N, or S367Q is preferred, and S367P is more preferred. As an amino acid substitution at F2196, F2196L or F2196M is preferred, and F2196L is more preferred.

In one particularly preferred embodiment, the variant of the present invention comprises the amino acid substitutions K213N, S367P, and F2196L in wild-type human factor VIII (hereinafter sometimes referred to as the "NPL mutation" after the amino acid residues after substitution). A variant having the NPL mutation has the characteristic of having high specific activity as compared with the wild type, provided that no other mutation or combination of mutations that cancels the effect thereof is present. As described above, a variant having high specific activity can achieve coagulation activity equal to or higher than that achieved when wild-type factor VIII is expressed at a high level, at an appropriate protein expression level that does not impair the long-term stability of the therapeutic effect, and is therefore useful for gene therapy for hemophilia A.

The variant of the present invention may have an amino acid residue at a position other than K213, S367, and F2196 substituted with another amino acid, in addition to at least one amino acid substitution selected from the group consisting of K213, S367, and F2196, as long as the characteristic of having high specific activity and/or secretory expression efficiency as compared with the wild type is retained. Examples of such substitution positions include R-5, P25, A28, L152, M217, W228, Q410, Y487, R489, F501, M539, I566, L603, I642, S727, A736, S1657, Q1659, E1661, I1668, D1681, R1776, H1859, A1993, H2007, N2019, K2085, K2207, F2275, S2296, V2314, Q2316, and M2321. Accordingly, in one embodiment, the variant of the present invention is one in which at least one of the amino acid residues described above is further substituted with another amino acid.

The other amino acids are not particularly limited as long as the human factor VIII variant obtained as a result of the amino acid substitution still has enhanced specific activity and/or secretory expression efficiency as compared with the wild type, but at least one amino acid substitution selected from the group consisting of R-5P, P25H, A28T, L152A, L152D, L152E, L152H, L152I, L152M, L152N, L152P, L152Q, or L152V, M217T, W228Q, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, K2207Q, F2275L, S2296A, V2314A, Q2316H, and M2321L is preferred.

More preferably, examples of the other amino acid substitutions include
(i) L152A, L152D, L152E, L152H, L152I, L152M, L152N, L152P, L152Q, or L152V, and/or
(ii) I1668F and D1681G.

One particularly preferred embodiment of the present invention includes a variant comprising, in addition to the NPL mutation, the amino acid substitution of (i) above (hereinafter sometimes referred to as an "XNPL mutation", and a case where L152 is substituted with proline (P) is sometimes referred to as a "PNPL mutation"). A variant having an XNPL mutation, including the PNPL mutation, may have, in addition to having high specific activity as compared with the wild type, the further characteristic of being expressed and secreted at a high level as compared with the wild type. Accordingly, in addition to the advantage imparted by the NPL mutation, the variant may have the further advantage that induction of endoplasmic reticulum stress is suppressed by improvement of secretion efficiency and long-term stability of the therapeutic effect of human factor VIII can be achieved.

Another preferred embodiment of the present invention includes a variant comprising, in addition to the NPL mutation or XNPL mutation, the amino acid substitutions of (ii) above (hereinafter sometimes referred to as the "FG mutation"). In a variant having the FG mutation, intracellular retention of the light chain is markedly suppressed as compared with corresponding human factor VIII not having either of the I1668F and D1681G mutations, and therefore further improvement may be observed in secretion efficiency and/or reconstitution of the heavy chain and light chain.

In one preferred embodiment, the variant of the present invention
has K213N as the amino acid substitution at K213,
further comprises M539L and, optionally, at least one amino acid substitution within the A2 domain selected from the group consisting of I566M, L603P, and I642V, and
further comprises K2207Q, Q2316H, and M2321L and, optionally, at least one amino acid substitution within the C2 domain selected from the group consisting of F2275L, S2296A, and V2314A.

By comprising at least the amino acid substitutions K213N, M539L, K2207Q, Q2316H, and M2321L, the characteristic of being expressed and secreted at a high level as compared with wild-type human factor VIII may be imparted even without comprising the NPL mutation. The secretory expression efficiency of the variant may be further enhanced by further comprising the FG mutation.

In another preferred embodiment, the variant of the present invention further comprises the amino acid substitutions L152P, Y487H, and L603P in addition to the NPL mutation, and optionally further comprises at least one amino acid substitution selected from the group consisting of S727P, Q1659E, H1859R, A1993V, H2007Q, N2019K, and K2085M. Since the variant comprises the PNPL mutation, it has high specific activity and is expressed and secreted at a high level as compared with the wild type.

In another preferred embodiment, the variant of the present invention further comprises the amino acid substitutions M539L, I566M, L603P, I642V, K2207Q, F2275L, S2296A, V2314A, Q2316H, and M2321L in addition to the NPL mutation. While the characteristic of high specific activity due to the NPL mutation may be attenuated by the variant having a further amino acid mutation comprising the above ten amino acid substitutions, its secretory expression efficiency is enhanced regardless of the presence or absence of an amino acid substitution at L152 (e.g., L152P) (that is, even without having an XNPL mutation). The secretory expression efficiency of the variant may be further enhanced by further comprising the FG mutation.

Preferred embodiments of the present invention include human factor VIII variants having the following amino acid substitutions (a) to (n) in wild-type human factor VIII.
(a) K213N, S367P and F2196L
(b) K213N, S367P, F2196L, I1668F and D1681G
(c) K213N, M539L, I566M, I642V, K2207Q, F2275L, Q2316H and M2321L
(d) K213N, M539L, I566M, L603P, I642V, F2196L, K2207Q, F2275L, Q2316H and M2321L
(e) K213N, M539L, I566M, I642V, F2196L, K2207Q, F2275L, S2296A, V2314A, Q2316H and M2321L
(f) K213N, M539L, I566M, I642V, I1668F, D1681G, F2196L, K2207Q, F2275L, S2296A, V2314A, Q2316H and M2321L
(g) L152P, K213N, S367P and F2196L
(h) L152P, K213N, S367P, Y487H, L603P and F2196L
(i) L152P, K213N, S367P, Y487H, L603P, S727P, Q1659E and F2196L
(j) L152P, K213N, S367P, Y487H, L603P, S727P, Q1659E, H1859R, A1993V, H2007Q, K2085M and F2196L
(k) K213N, S367P, M539L, I566M, L603P, I642V, F2196L, K2207Q, F2275L, S2296A, V2314A, Q2316H and M2321L
(l) P25H, A28T, K213N, M217T, S367P, Q410L, Y487H, F501M, M539L, I566M, L603P, I642V, S727P, A736V, S1657P, Q1659E, E1661K, I1668F, D1681G, R1776K, N2019K, F2196L, K2207Q, F2275L, S2296A, V2314A, Q2316H and M2321L
(m) R-5P, P25H, A28T, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, N2019K, F2196L, K2207Q, F2275L, S2296A, V2314A, Q2316H and M2321L
(n) R-5P, P25H, A28T, L152P, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, F2196L, K2207Q, F2275L, S2296A, V2314A, Q2316H and M2321L

Since the B domain of human factor VIII contains no regions that interact with various proteins and the B domain does not contribute to cofactor activity in circulating blood, coagulation factor activity is retained even when the B domain is deleted. Accordingly, the variant of the present invention may have the B domain deleted. "Having the B domain deleted" encompasses not only a case where the amino acid sequence of the B domain (positions 741 to 1648) is completely deleted, but also a case where an amino acid sequence of about 1 to 15 amino acid residues at the N terminus and/or C terminus of the B domain is retained between the a2 region and the a3 region. A B-domain-deleted (BDD) human factor VIII variant has an advantage when an AAV vector is used in gene therapy for hemophilia A. An AAV vector has a small size of 4.7 kbp for a nucleic acid that can be carried therein, but full-length human factor VIII consists of 2,351 amino acids, and therefore the coding region alone exceeds 7 kbp and cannot be carried in an AAV vector. On the other hand, the coding sequence of BDD human factor VIII is less than 4.4 kbp, and the entire expression cassette, including a promoter sequence and the like, also falls within the packaging size of an AAV vector.

In one preferred embodiment, the variant of the present invention has positions 744 to 1637 of the B domain deleted (corresponding to positions 763 to 1656 in the amino acid sequence represented by SEQ ID NO: 1) (that is, S743 and Q1638 are linked; sometimes referred to as "FVIII SQ" or "SQ"). BDD human factor VIIIs that lead to efficient processing of human factor VIII, such as deletion of the B domain leaving portions of both ends of the B domain other than FVIII SQ, or substitution of the B domain with three to four arginine (R) residues, are known (e.g., Lind et al., Eur. J. Biochem. 232, 19-27 (1995)) and can also be used in the present invention.

On the other hand, since the B domain is known to be involved in intracellular transport of factor VIII, the variant of the present invention may comprise all or part of the B domain for the purpose of improving secretion efficiency. For example, when a lentiviral (LV) vector or adenoviral (AdV) vector having a larger packaging size is used in gene therapy, when mRNA encoding the variant of the present invention is used as an mRNA medicament, or when the variant of the present invention is recombinantly produced and used as a protein preparation, it is also preferable to use a variant comprising the B domain.

The variant of the present invention may be one that is expressed in the body of an animal to be treated by administering a nucleic acid encoding the same to the animal in vivo or ex vivo, or may be a recombinant protein obtained by introducing the nucleic acid into an appropriate host cell, causing the nucleic acid to be expressed, and culturing the host cell. In either case, since the variant of the present invention is produced based on a nucleic acid encoding the same, the present invention also provides a nucleic acid encoding any of the variants of the present invention described above.

The "nucleic acid encoding a human factor VIII variant" used in the present invention may be DNA or RNA, or may be a DNA/RNA chimera. In addition, the nucleic acid may be double-stranded or single-stranded. When it is double-stranded, it may be double-stranded DNA, double-stranded RNA, or a DNA:RNA hybrid. When it is single-stranded, it may be a sense strand (i.e., a coding strand) or an antisense strand (i.e., a noncoding strand). The type of nucleic acid can be appropriately selected according to its intended use (the type of vector to be used and the like), but is preferably DNA, and more preferably double-stranded DNA. In addition, the nucleic acid may be a physiologically acceptable salt with an acid or base, and, for example, a physiologically acceptable acid addition salt is preferred. As such salts, among others, salts with inorganic acids (e.g., hydrochloric acid, phosphoric acid, hydrobromic acid, and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, and benzenesulfonic acid), and the like are used.

Examples of DNA encoding a human factor VIII variant include genomic DNA, cDNA (cRNA) derived from human or other mammalian cells or tissues, synthetic DNA (RNA), and the like.

More specifically, examples of the "nucleic acid encoding the variant of the present invention" include any nucleotide sequence encoding a modified amino acid sequence having any of the amino acid substitutions described above in the amino acid sequence of wild-type human factor VIII represented by SEQ ID NO: 1, or in the amino acid sequence of a natural allelic variant or genetic polymorphism (e.g., an SNP or the like) of a protein consisting of the amino acid sequence. Preferably, the nucleotide sequence is a sequence codon-optimized for expression in a host cell to be used, preferably a human cell. At the time of gene expression, an increase in the amount of protein expression can be expected by converting the nucleotide sequence into codons frequently used in the host organism. As data on codon usage frequency in the host to be used, for example, the Codon Usage Database made publicly available on the website of the Kazusa DNA Research Institute (http://www.kazusa.or.jp/codon/index.html) can be used, or literature describing codon usage frequency in each host may be referred to. Alternatively, as used in the Examples described below, codon optimization can also be performed using a codon optimization algorithm known per se (e.g., GeneArt Codon Optimizer). In such an algorithm, in addition to the frequency of use in the host, multiple parameters such as GC content, removal of destabilizing RNA elements, removal of cryptic splice sites, removal of intragenic polyA sites, removal of repetitive sequences, avoidance of RNA secondary structures, and removal of IRESs may be taken into consideration. DNA encoding a desired variant of the present invention can be constructed by chemically synthesizing a DNA strand having the codon-optimized sequence obtained as described above, or by connecting chemically synthesized, partially overlapping short oligo-DNA strands using a PCR method or Gibson Assembly method.

In one preferred embodiment, examples of the nucleic acid encoding the variant of the present invention include a nucleic acid consisting of the nucleotide sequence represented by SEQ ID NO: 2, which encodes a variant having the amino acid substitutions of (n) above, namely, R-5P, P25H, A28T, L152P, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, F2196L, K2207Q, F2275L, S2296A, V2314A, Q2316H, and M2321L in wild-type human factor VIII consisting of the amino acid sequence represented by SEQ ID NO: 1, and which has been codon-optimized for humans.

A nucleotide sequence codon-optimized for a target host (a human in use in gene therapy or an mRNA medicament for hemophilia A) may have one or more CpG sequences within the sequence. On the prediction that CpG sequences may induce an immune response in the host and inhibit sustained expression of the transgene, expression efficiency in a host cell can be markedly increased by performing base substitutions that remove these CpG sequences without changing the amino acid sequence of the variant, and then performing codon optimization again. For example, a variant-encoding nucleic acid consisting of the nucleotide sequence represented by SEQ ID NO: 2 and codon-optimized for humans can be modified so as not to contain CpG sequences without changing the amino acid sequence encoded thereby. An example of such a CpG-removed and re-codon-optimized nucleotide sequence is the nucleotide sequence represented by SEQ ID NO: 3.

The improvement in expression efficiency by removal of CpG sequences produces the effect not only in the above specific human factor VIII variant but also in nucleic acids encoding any variant and, further, wild-type human factor VIII. Accordingly, the present invention also provides a human factor VIII-encoding nucleic acid consisting of a nucleotide sequence obtained by modifying a nucleic acid encoding human factor VIII codon-optimized for a target host, preferably humans, so as not to contain CpG sequences without changing the amino acid sequence encoded thereby, and then further performing codon optimization again. For example, the nucleotide sequence represented by SEQ ID NO: 4 is an example of a nucleotide sequence codon-optimized for humans and encoding wild-type human factor VIII consisting of the amino acid sequence represented by SEQ ID NO: 1. An example of a nucleotide sequence obtained by modifying the sequence so as not to contain CpG sequences without changing the amino acid sequence encoded thereby and then further performing codon optimization again for humans is the nucleotide sequence represented by SEQ ID NO: 5.

A nucleic acid encoding the variant of the present invention, or a human factor VIII-encoding nucleic acid having enhanced expression efficiency in a host cell by codon optimization and removal of CpG sequences (hereinafter these may collectively be referred to as "the nucleic acid of the present invention"), can be used to construct an expression vector capable of expression in a liver cell by being linked downstream of a promoter functional in a liver cell and inserted into a vector.

The liver is composed of hepatic parenchymal cells (hepatocytes), which perform the major functions of the liver, such as bile production and substance metabolism, and hepatic non-parenchymal cells, such as liver sinusoidal endothelial cells, Kupffer cells, hepatic stellate cells, pit cells, bile duct epithelial cells, and mesothelial cells. In the present specification, the "liver cell" means one or more types of the above cell groups constituting the liver, including cancer cells and cell lines derived therefrom.

In the present specification, the "promoter functional in a liver cell" means a promoter capable of inducing transcription of a gene linked downstream thereof (a nucleic acid encoding a protein) in a liver cell. In consideration of the fact that a preferred use of the present invention is in vivo gene therapy, it is desirable that the promoter functions specifically in a liver cell (induces transcription of the downstream gene). Here, the "specifically in a liver cell" and "liver-specific" are used with the intention of encompassing not only a case where expression is localized only in liver cells, but also a case where expression in liver cells is significantly higher than expression in cells of other organs or tissues. Even when gene expression is induced in cells other than liver cells, substantially liver-specific gene expression can be achieved, for example, to an extent that does not cause undesirable side effects, by combining the promoter with a vector having high organ, tissue, or cell tropism (for example, various serotypes of viral vectors).

The promoter may further comprise other regulatory sequences (e.g., an endogenous proximal or distal enhancer sequence, an enhancer sequence derived from another gene, or the like), as long as it comprises a minimum nucleotide sequence that exerts basal transcriptional activity.

The promoter functional in a liver cell is not particularly limited as long as it is capable of inducing transcription of a downstream gene in a liver cell, but is preferably a promoter of a gene highly expressed in the liver, and more preferably a liver-specific promoter. Here, "liver-specific" has the same meaning as described above. Examples of promoters functional in liver cells that can be used in the expression vector of the present invention include, but are not limited to, a transthyretin (TTR) promoter, an α1-antitrypsin (AAT) promoter, an albumin promoter, an α-fetoprotein promoter, and a thyroxine-binding globulin promoter. In one preferred embodiment, for example, when use in gene therapy for hemophilia A using an AAV vector is intended, a TTR promoter or AAT promoter can be used as the promoter functional in liver cells. In another preferred embodiment, a chimeric promoter of a human AAT promoter and the hepatic control region (HCR) of the Apo E/C1 gene (HCRhAAT) can also be used as the promoter functional in liver cells.

A promoter functional in liver cells can be obtained by a method known per se based on sequence information of the gene (genomic DNA) from which each promoter is derived. Such sequence information is registered in publicly available databases such as NCBI, EMBL, FASTA, and DDBJ, and is also described in the literature. A person skilled in the art can readily access the sequence information.

When the nucleotide sequence of a promoter functional in liver cells contains one or more CpG sequences, in order to enhance the expression efficiency in a host cell of the nucleic acid of the present invention linked downstream thereof, it is desirable to modify the promoter so as to remove the CpG sequences, in the same manner as in the case of the nucleic acid of the present invention. For example, the wild-type mouse TTR promoter consists of the nucleotide sequence represented by SEQ ID NO: 6, but a modified promoter consisting of the nucleotide sequence represented by SEQ ID NO: 7, from which the CpG sequences in the sequence have been removed, can be used.

The linkage of a promoter functional in liver cells with the nucleic acid of the present invention can be performed by a method known per se in the art. For example, when each fragment of the promoter and the nucleic acid has blunt ends, the two fragments can be ligated using DNA ligase. Alternatively, any adapter sequence may be added to the ends of the two fragments, the fragments may be treated with an appropriate restriction enzyme to generate cohesive ends, and then the two fragments may be ligated.

In a preferred embodiment, the nucleic acid of the present invention linked downstream of a promoter functional in liver cells is preferably inserted into a vector in the form of an expression cassette in which a transcription termination signal functional in liver cells, namely, a polyadenylation signal (e.g., an SV40 polyadenylation signal), is further linked downstream thereof.

For example, an expression cassette in which (ii) a human factor VIII-encoding sequence (SEQ ID NO: 3) that encodes a variant having the amino acid substitutions of (n) above, namely, R-5P, P25H, A28T, L152P, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, F2196L, K2207Q, F2275L, S2296A, V2314A, Q2316H, and M2321L in wild-type human factor VIII, and that has been codon-optimized for humans, further had CpG sequences removed, and has been codon-optimized again; and (iii) an SV40 polyadenylation signal sequence are linked downstream of (i) a mouse TTR promoter modified so as not to contain CpG sequences (SEQ ID NO: 7), may preferably be used. An example of such expression cassette is a nucleic acid consisting of the nucleotide sequence represented by SEQ ID NO: 8 (4814 bp). The nucleic acid contains a 5'-upstream sequence consisting of 17 nucleotides (positions 283 to 299) between the promoter sequence of SEQ ID NO: 7 (positions 1 to 282) and the coding sequence of SEQ ID NO: 3 (positions 300 to 4673), and the secretory expression efficiency of human factor VIII can be improved by deleting 11 nucleotides at positions 283 to 293 therefrom to truncate the same. The nucleotide sequence (4803 bp) in which the 11 nucleotides have been deleted from the nucleotide sequence of SEQ ID NO: 8 is shown in SEQ ID NO: 9. In addition, in SEQ ID NO: 9, the nucleotide sequence (4790 bp) in which 13 nucleotides on the 3'-terminal side have been deleted from the SV40 polyadenylation signal sequence (positions 4663 to 4803) is shown in SEQ ID NO: 10, and the nucleotide sequence (4768 bp) in which 35 nucleotides on the 3'-terminal side have been deleted is shown in SEQ ID NO: 11. By these modifications (truncating) of the polyadenylation signal, the secretory expression efficiency of human factor VIII may be further improved.

The vector into which the nucleic acid of the present invention is inserted is not particularly limited as long as it is generally used for gene therapy. For example, viral vectors such as an AAV vector, an LV vector, a retroviral vector, an AdV vector, a Sindbis virus vector, a rabies virus vector, a Sendai virus vector, and a herpes simplex virus vector, and non-viral vectors such as a plasmid for animal cells can be used. From the viewpoints of high gene introduction and expression efficiency, capability of introduction also into non-dividing cells, capability of long-term expression of a transgene, and the like, an AAV vector and an LV vector are preferred. From the viewpoints of a low frequency of chromosomal integration, absence of the risk of insertional mutation, low immunogenicity, and high safety, it is more preferable to use an AAV vector. An AAV vector is diluted in dividing cells along with cell proliferation, but adult hepatocytes hardly divide, and therefore expression of the transgene can be retained for a long period of time. However, since the size of a gene that can be carried is as small as 4.7 kbp, depending on the size of the nucleic acid to be introduced, use of an LV vector or AdV vector capable of carrying a larger insert may also be preferred.

For example, when an AAV vector is used as the vector, it is desirable to use a vector derived from a serotype having high liver tropism. Examples of serotypes having liver tropism include AAV1, AAV2, AAV3, AAV5, AAV6, AAV6.2, AAV7, AAV8, AAV9, AAVrh10, AAV-DJ, and AAV-DJ/8. Procedures for constructing a vector are described below using an AAV vector as an example. However, a person skilled in the art can also readily construct a desired expression vector for another viral vector or non-viral vector (e.g., a plasmid vector) using a method known per se [for example, Current Protocols in Molecular Biology, F. Ausubel et al., eds. (1994), John Wiley & Sons, Inc.; Molecular Cloning (A Laboratory Manual), 3rd ed., Volumes 1-3, Joseph Sambrook & David W. Russell, eds., Cold Spring Harbor Laboratory Press (Cold Spring Harbor, New York) (2001); Culture of Animal Cells: A Manual of Basic Technique, R. Freshney, ed., 2nd ed. (1987), Wiley-Liss; Frank L. Graham, Manipulation of Adenovirus Vector, Chapter 11, pp. 109-128; E. J. Murray, ed., Methods in Molecular Biology, Vol. 7, Gene Transfer and Expression Protocols (1991); Chen, S.-H. et al., Combination Gene Therapy for Liver Metastases of Colon Carcinoma In Vivo, Proc. Natl. Acad. Sci. USA (1995) 92, 2477-2581; and the like].

In preparation of an AAV expression vector, the synthetic promoter of the present invention is first inserted, together with the 5'- and 3'-ITRs of AAV, between the two ITRs in a plasmid capable of being amplified in an appropriate host cell (e.g., Escherichia coli, Bacillus subtilis, yeast, or the like). Examples of the plasmid include plasmids derived from Escherichia coli (e.g., pBR322, pBR325, pUC12, and pUC13), plasmids derived from Bacillus subtilis (e.g., pUB110, pTP5, and pC194), and plasmids derived from yeast (e.g., pSH19 and pSH15). As the AAV ITRs, ITRs derived from the target serotype may be used, but ITRs derived from AAV2 can generally be commonly used. Only the synthetic promoter of the present invention may be inserted between the two ITR sequences, but it is desirable to insert a polyadenylation signal (e.g., an SV40 polyadenylation signal) downstream thereof, and a woodchuck hepatitis virus post-transcriptional regulatory element (WPRE) may further be inserted between the synthetic promoter and the polyadenylation signal. A truncated WPRE (0.25 kb; Mol. Brain, 7:17, 2014) may also be used. By inserting one or more restriction enzyme recognition sites, preferably a multiple cloning site (MCS), between the promoter of the present invention and the polyadenylation signal (or, when WPRE is included, between the promoter and WPRE), insertion of the nucleic acid of the present invention (construction of a human factor VIII expression cassette) can be facilitated.

By inserting the nucleic acid of the present invention to be expressed in liver cells between the promoter and the polyadenylation signal of the obtained expression plasmid for an AAV vector (or, when WPRE is included, between the promoter and WPRE), a human factor VIII expression cassette is constructed in the plasmid.

The expression vector may further contain, as desired, a 5'-UTR capable of functioning in the host downstream of the promoter, and a 3'-UTR capable of functioning in the host downstream of the DNA encoding human factor VIII. The expression vector may also contain an enhancer, a splicing signal, and the like. As each of these constituent elements, an element known per se can be used.

An AAV vector can be produced from an AAV vector expression plasmid containing the nucleic acid of the present invention downstream of a promoter functional in liver cells by a method known per se, for example, a plasmid transfection method, a recombinant baculovirus method, a recombinant herpesvirus vector method, a yeast method, or the like. For example, in the plasmid transfection method, the AAV vector expression plasmid, a plasmid containing the Rep and Cap genes of AAV, and a pHelper plasmid containing the adenovirus-derived E2A, E4orf6, and VA RNA genes can be transfected into HEK293 cells or the like to produce AAV viral particles. The Rep gene does not need to be derived from the target serotype, and a Rep gene derived from AAV2 is generally used. On the other hand, the Cap gene needs to be derived from the target serotype, and the cell tropism of the AAV serotype is determined by the capsid protein encoded by the Cap gene. That is, in the expression vector of the present invention targeting liver cells, a Cap gene derived from AAV1, AAV2, AAV3, AAV5, AAV6, AAV6.2, AAV7, AAV8, AAV9, AAVrh10, AAV-DJ, AAV-DJ/8, or the like having liver tropism can be used as the Cap gene.

On the other hand, when a non-viral vector is used as the expression vector of the present invention, the expression vector can be introduced using a polymer carrier such as a poly-L-lysine-nucleic acid complex or by encapsulating it in a liposome. A liposome is a capsule composed of phospholipids and having a particle size of several tens to several hundreds of nanometers, and a plasmid vector can be enclosed therein.

An expression vector containing the nucleic acid of the present invention downstream of a promoter functional in liver cells can treat a disease in which human factor VIII exerts a therapeutic effect, particularly hemophilia A, by being administered to a subject suffering from the disease. Accordingly, the present invention also provides a gene therapeutic agent for hemophilia A comprising the expression vector of the present invention.

In the gene therapeutic agent of the present invention, the expression vector of the present invention may be used as the active substance as it is, but, as necessary, it may also be used as a medicament after being mixed together with a pharmacologically acceptable carrier and made into various formulation forms such as an injection.

Here, as the pharmacologically acceptable carrier, various organic or inorganic carrier substances conventionally used as formulation materials are used and are incorporated as a solvent, solubilizing agent, suspending agent, isotonic agent, buffer, soothing agent, or the like in a liquid formulation. In addition, formulation additives such as a preservative, antioxidant, and coloring agent may also be used as necessary.

Suitable examples of the solvent include water for injection, physiological saline, Ringer's solution, alcohol, propylene glycol, polyethylene glycol, sesame oil, corn oil, olive oil, cottonseed oil, and the like.

Suitable examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, and the like.

Suitable examples of the suspending agent include surfactants such as stearyltriethanolamine, sodium lauryl sulfate, lauryl aminopropionic acid, lecithin, benzalkonium chloride, benzethonium chloride, and glycerol monostearate, hydrophilic polymers such as polyvinyl alcohol, polyvinylpyrrolidone, sodium carboxymethylcellulose, methylcellulose, hydroxymethylcellulose, hydroxyethylcellulose, and hydroxypropylcellulose, polysorbates, polyoxyethylene hydrogenated castor oil, and the like.

Suitable examples of the isotonic agent include sodium chloride, glycerin, D-mannitol, D-sorbitol, glucose, and the like.

Suitable examples of the buffer include buffer solutions such as phosphates, acetates, carbonates, and citrates.

Suitable examples of the soothing agent include benzyl alcohol and the like.

Suitable examples of the preservative include paraoxybenzoic acid esters, chlorobutanol, benzyl alcohol, phenethyl alcohol, dehydroacetic acid, sorbic acid, and the like.

Suitable examples of the antioxidant include sulfites, ascorbates, and the like.

Suitable examples of the coloring agent include water-soluble edible tar dyes (e.g., food Red Nos. 2 and 3, food Yellow Nos. 4 and 5, food Blue Nos. 1 and 2, and other food dyes), water-insoluble lake dyes (e.g., aluminum salts of the above water-soluble edible tar dyes), natural pigments (e.g., β-carotene, chlorophyll, red iron oxide, and the like), and the like.

Examples of the dosage form of the pharmaceutical composition include parenteral preparations such as injections (e.g., subcutaneous injections, intravenous injections, intramuscular injections, intraperitoneal injections, and the like) and infusions.

The gene therapeutic agent of the present invention can be produced by a method conventionally used in the field of pharmaceutical formulation technology, for example, a method described in the Japanese Pharmacopoeia or the like. The content of the viral vector, which is the active ingredient in the formulation, varies depending on the dosage form, the dose of the active ingredient, and the like, but is, for example, about 0.1 to 100% by weight. The viral titer can be appropriately adjusted so as to be, for example, about 1×10¹⁰ to 10¹³ vp/mL, but is not limited to this range.

Formulations suitable for parenteral administration (e.g., intravenous injection, subcutaneous injection, intramuscular injection, intraperitoneal administration, and the like) include aqueous and non-aqueous isotonic sterile injection solutions, which may contain an antioxidant, buffer solution, bacteriostatic agent, isotonic agent, and the like. Aqueous and non-aqueous sterile suspension preparations are also included, which may contain a suspending agent, solubilizing agent, thickening agent, stabilizing agent, preservative, and the like. The most suitable dosage form in the present invention is an injection solution.

The dose of the formulation varies depending on the type of vector, promoter activity, administration route, severity of the disease, animal species to be administered, drug acceptability, body weight, age, and the like. For example, when an AAV vector (e.g., AAV8) using an HCRhAAT promoter as a basal promoter is used as a human factor VIII expression vector for hemophilia A, in the case of systemic administration, particularly administration of the AAV vector through a peripheral vein, it can be administered, for example, at a single dose of about 5×10¹² to about 5×10¹³ vp/kg body weight. Since deaths due to severe liver injury have been reported in a high-dose administration group (3×10¹⁴ vp/kg body weight) in a recent clinical trial of an AAV vector (Audentes Therapeutics. Letter to the MTM disease community. https://myotubulartrust.org/audentes-therapeutics-letter-23-june-2020/; Hum Gene Ther 2020; 31: 695-696), it is desirable to administer the vector at a dose of 10¹⁴ vp/kg body weight or less. For example, in one preferred embodiment, the gene therapeutic agent of the present invention can be systemically administered at a dose, as the amount of the AAV vector, of about 1×10¹⁰ to about 5×10¹³ vp/kg body weight, preferably about 1×10¹¹ to about 1×10¹³ vp/kg body weight, more preferably about 5×10¹¹ to about 5×10¹² vp/kg body weight or about 1×10¹² to about 1×10¹³ vp/kg body weight.

The present invention also provides a method for expressing human factor VIII in liver cells, comprising introducing a gene therapeutic agent containing the expression vector of the present invention or the nucleic acid of the present invention into liver cells in vitro. By transplanting liver cells transfected with the gene therapeutic agent of the present invention into a subject having hemophilia A, an ex vivo treatment method for the disease can be provided. Accordingly, the present invention also provides a medicament (cell preparation) containing, as an active ingredient, liver cells transfected with the gene therapeutic agent of the present invention, particularly a therapeutic agent for hemophilia.

When the nucleic acid of the present invention is single-stranded RNA, the nucleic acid can be formulated in the form of an mRNA medicament. For example, the mRNA can be obtained by introducing the expression vector into an appropriate host (e.g., a mammalian cell), culturing the host, recovering mRNA using a method known per se (e.g., an LiCl method), and purifying the mRNA encoding human factor VIII.

Alternatively, the mRNA can also be obtained by excising, from the expression vector, a region including the human factor VIII coding sequence (CDS) as well as the 5'- and 3'-UTRs, and converting the region, as a template, into mRNA encoding the present human factor VIII using an in vitro transcription system known per se. More specifically, a fragment of the mRNA-coding region to which a phage (T7, T3, SP6, or the like) promoter is linked is obtained by excising the mRNA-coding region using an appropriate restriction enzyme and ligating the phage promoter to the 5' end thereof, or by performing PCR using the expression vector as a template and a primer containing the phage promoter sequence. Using the obtained DNA fragment as a template, a phage (T7, T3, SP6, or the like) RNA polymerase can be reacted therewith to synthesize mRNA encoding human factor VIII in vitro. At this time, by adding a triphosphate of pseudouridine (Ψ) or N1-methylpseudouridine (N1mΨ) to the reaction solution instead of UTP as an RNA monomer (NTP), the obtained mRNA contains Ψ or N1mΨ instead of U. As a result, attack by innate immunity against the mRNA can be avoided, and the stability and translation efficiency of the mRNA are improved. Similarly, modified NTPs having base substitutions that have been reported to avoid innate immunity may also be used for the other NTPs (for example, inosine triphosphate instead of ATP, 5-methylcytidine triphosphate instead of CTP, and the like).

It is also desirable to add a 5'-cap structure and a poly(A) tail, which are necessary for stabilization of mRNA and efficient translation. The 5'-cap structure can be added as a Cap 0 structure using a capping enzyme after synthesis of the mRNA and can further be converted into a Cap 1 structure by mRNA 2'-O-methyltransferase. Alternatively, the transcription reaction and 5'-capping can be performed simultaneously by adding an RNA cap analog (e.g., 3'-O-Me-m7G(5')ppp(5')G, m7G(5')ppp(5')G, 3'-O-Me-m7G(5')ppp(5')A, m7G(5')ppp(5')A, or the like) to the transcription reaction solution. Poly(A) tailing can also be performed by adding a poly(A) tail to the 3' end using poly(A) polymerase after mRNA synthesis, or can be performed simultaneously with the transcription reaction by adding a poly(A) sequence in advance to the template for the transcription reaction. From the mRNA obtained as described above, the template DNA can be removed with DNase I, and the mRNA can be purified by, for example, an LiCl method or the like.

In order to promote introduction of the nucleic acid of the present invention into target cells, a reagent for nucleic acid introduction may be used. As the reagent for nucleic acid introduction, atelocollagen; liposomes; nanoparticles; cationic lipids such as lipofectin, lipofectamine, DOGS (Transfectam), DOPE, DOTAP, DDAB, DHDEAB, HDEAB, polybrene, or poly(ethyleneimine) (PEI); and the like can be used.

In one embodiment, the nucleic acid of the present invention may be a pharmaceutical composition in which the nucleic acid is encapsulated in a liposome. A liposome is a fine closed vesicle having an internal phase surrounded by one or more lipid bilayers, and can generally retain a water-soluble substance in the internal phase and a lipid-soluble substance within the lipid bilayer. Here, when the "encapsulated" is used, the nucleic acid of the present invention may be retained in the internal phase of the liposome or may be retained within the lipid bilayer. The liposome used in the present invention may be unilamellar or multilamellar, and the particle diameter can be appropriately selected, for example, within the range of 10 to 1,000 nm, and preferably 50 to 300 nm. In consideration of deliverability to a target tissue, the particle diameter may be, for example, 200 nm or less, and preferably 100 nm or less.

Examples of methods for encapsulating a water-soluble compound such as a polynucleotide in a liposome include a lipid film method (vortex method), reverse-phase evaporation method, detergent removal method, freeze-thaw method, remote loading method, and the like, but are not limited thereto, and any known method can be appropriately selected.

In a preferred embodiment, the nucleic acid of the present invention uses lipid nanoparticles as a carrier and is encapsulated in the particles.

In the present specification, the "lipid nanoparticle" (Lipid Nano Particle; sometimes abbreviated herein as "LNP") means a particle having a membrane structure in which hydrophilic groups of amphiphilic lipids are arranged toward the aqueous phase side of an interface and having a particle diameter of less than 1 µm, and the "amphiphilic lipid" means a lipid having both a hydrophilic group and a hydrophobic group.

The particle diameter of the lipid nanoparticles used in the present invention is preferably 10 nm to 500 nm, and more preferably 30 nm to 300 nm. The particle diameter can be measured using, for example, a particle size distribution analyzer such as Zetasizer Nano (Malvern). The particle diameter of the lipid nanoparticles can be appropriately adjusted by the method for producing the lipid nanoparticles. In the present specification, the "particle diameter" means an average particle diameter (Z-average) measured by a dynamic light scattering method.

Examples of amphiphilic lipids include cationic lipids, ionizable lipids, phospholipids, PEG lipids, and the like. In the present specification, the "cationic lipid" means a lipid having a hydrophilic group that is constantly positively charged. In the present specification, the "ionizable lipid" means a lipid that is neutral at physiological pH but is protonated and positively charged at low pH. In the present specification, the "PEG" means polyethylene glycol, and the "PEG lipid" means a lipid modified with PEG, namely, a lipid to which PEG is bound.

In a preferred embodiment, examples of the lipid nanoparticle in which the nucleic acid of the present invention is encapsulated include a lipid nanoparticle comprising
(A) a cationic lipid or an ionic lipid,
(B) a phospholipid,
(C) a steroid, and
(D) a PEG lipid.

Examples of each component lipid of (A) to (D) include known lipids ordinarily used in lipid nanoparticles, and a person skilled in the art can readily select appropriate types of lipids and compositions thereof.

The present invention also provides a host cell into which an expression vector containing the nucleic acid of the present invention has been introduced; a method for producing human factor VIII or a variant thereof, comprising culturing the host cell and recovering human factor VIII or the variant thereof from the resulting culture; and a medicament (recombinant protein preparation) comprising the human factor VIII variant obtained by the method, particularly a therapeutic agent for hemophilia A.

An expression vector for production of recombinant human factor VIII can be produced by linking the nucleic acid of the present invention (i.e., a nucleic acid encoding the variant of the present invention, or a high-expression-type human factor VIII-encoding nucleic acid) downstream of a promoter in an appropriate expression vector. As the expression vector, plasmids derived from Escherichia coli (e.g., pBR322, pBR325, pUC12, and pUC13); plasmids derived from Bacillus subtilis (e.g., pUB110, pTP5, and pC194); plasmids derived from yeast (e.g., pSH19 and pSH15); insect cell expression plasmids (e.g., pFast-Bac); animal cell expression plasmids (e.g., pA1-11, pXT1, pRc/CMV, pRc/RSV, and pcDNAI/Neo); bacteriophages such as λ phage; insect virus vectors such as baculoviruses (e.g., BmNPV and AcNPV); animal virus vectors such as retroviruses, lentiviruses, vaccinia viruses, adenoviruses, adeno-associated viruses, and herpesviruses; and the like are used.

The promoter may be any promoter appropriate for the host used for expression of the gene. For example, when the host is an animal cell, an SRα promoter, SV40 promoter, LTR promoter, CMV (cytomegalovirus) promoter, RSV (Rous sarcoma virus) promoter, MoMuLV (Moloney murine leukemia virus) LTR, HSV-TK (herpes simplex virus thymidine kinase) promoter, or the like is used. Also in other hosts, a promoter known per se can be appropriately selected.

As the expression vector, in addition to the above, one containing, as desired, an enhancer, splicing signal, polyadenulation signal, selection marker, SV40 replication origin (hereinafter sometimes abbreviated as "SV40 ori"), or the like can be used. Examples of the selection marker include a dihydrofolate reductase gene, an ampicillin resistance gene, a neomycin resistance gene, and the like.

Human factor VIII or a variant thereof can be produced by transforming a host with an expression vector containing the nucleic acid of the present invention and culturing the resulting transformant. As the host, for example, bacteria belonging to the genus Escherichia, bacteria belonging to the genus Bacillus, yeast, insect cells, insects, animal cells, and the like are used. As mammalian cells, for example, monkey COS-7 cells, monkey Vero cells, Chinese hamster ovary cells (hereinafter abbreviated as "CHO cells"), dhfr gene-deficient CHO cells (hereinafter abbreviated as "CHO (dhfr-) cells"), mouse L cells, mouse AtT-20 cells, mouse myeloma cells, rat GH3 cells, human FL cells, HeLa cells, HepG2 cells, HEK293 cells, and the like are used. Also for other hosts, cells known per se can each be appropriately selected.

Transformation can be performed according to a known method depending on the type of host. Animal cells can be transformed, for example, according to the methods described in Cell Technology, Separate Volume 8, New Cell Technology Experimental Protocols, 263-267 (1995) (published by Shujunsha), and Virology, Vol. 52, 456 (1973).

Culturing of the transformant can be performed according to a known method depending on the type of host. For example, as a medium for culturing a transformant in which the host is an animal cell, minimum essential medium (MEM), Dulbecco's modified Eagle's medium (DMEM), RPMI 1640 medium, Medium 199, Ham's F-12 medium, or the like containing about 5 to about 20% fetal bovine serum is used. The pH of the medium is preferably about 6 to about 8. Culturing is ordinarily performed at about 30°C to about 40°C for about 15 to about 60 hr. Aeration or stirring may be performed as necessary.

In the manner described above, human factor VIII can be produced intracellularly or extracellularly in the transformant.

Human factor VIII can be separated and purified from a culture obtained by culturing the transformant according to a method known per se. As such methods, a method utilizing solubility, such as salting-out or solvent precipitation; a method mainly utilizing a difference in molecular weight, such as dialysis, ultrafiltration, gel filtration, or SDS-polyacrylamide gel electrophoresis; a method utilizing a difference in charge, such as ion-exchange chromatography; a method utilizing specific affinity, such as affinity chromatography; a method utilizing a difference in hydrophobicity, such as reversed-phase high-performance liquid chromatography; a method utilizing a difference in isoelectric point, such as isoelectric focusing; and the like are used. These methods may also be appropriately combined.

When the human factor VIII thus obtained is in a free form, the free form can be converted into a salt by a method known per se or a method analogous thereto, and when the human factor VIII is obtained as a salt, the salt can be converted into a free form or another salt by a method known per se or a method analogous thereto.

Human factor VIII has low toxicity and can be administered as it is as a liquid preparation, or as a pharmaceutical composition in an appropriate dosage form, parenterally to a human or another mammal (for example, intravascular administration (intravenous administration, intra-arterial administration, or the like), subcutaneous administration, intradermal administration, intraperitoneal administration, intramuscular injection, local administration, or the like).

As a composition for parenteral administration, for example, an injection, suppository, or the like is used, and the injection may encompass dosage forms such as an intravenous injection, subcutaneous injection, intradermal injection, intramuscular injection, and intravenous drip injection. Such an injection can be prepared according to a known method. As a method for preparing an injection, for example, the injection can be prepared by dissolving, suspending, or emulsifying one or more active ingredients in a sterile aqueous liquid or oily liquid ordinarily used for injections. As the aqueous liquid for injection, for example, physiological saline, an isotonic solution containing glucose or another auxiliary agent, or the like is used, and may be used in combination with an appropriate solubilizing agent, for example, an alcohol (e.g., ethanol), a polyalcohol (e.g., propylene glycol or polyethylene glycol), a nonionic surfactant [e.g., polysorbate 80 or HCO-50 (polyoxyethylene (50 mol) adduct of hydrogenated castor oil)], or the like. As the oily liquid, for example, sesame oil, soybean oil, or the like is used, and benzyl benzoate, benzyl alcohol, or the like may be used in combination as a solubilizing agent. The prepared injection solution is preferably filled into an appropriate ampoule. A suppository used for rectal administration may be prepared by mixing the active ingredient with an ordinary suppository base.

The above pharmaceutical composition for parenteral use is conveniently prepared in a dosage-unit form adapted to the dose of the active ingredient. Examples of such dosage-unit forms include a tablet, pill, capsule, injection (ampoule), and suppository, and ordinarily contain 100 to 5,000 units, preferably 250 to 3,000 units, per dosage-unit form.

The dose of human factor VIII varies depending on the subject to be administered, symptoms, administration route, and the like. For example, when used for treatment of hemophilia A, it is convenient to administer, as a single dose, ordinarily about 10 to 100 units/kg body weight, preferably about 20 to 50 units/kg body weight, preferably about two to three times per week, by intravenous or intraperitoneal administration. Also in the case of another parenteral administration, an amount corresponding thereto can be administered. When the symptoms are particularly severe, the dose may be increased according to the symptoms.

The present invention is described in more detail below with reference to examples; however, these are merely illustrative and do not limit the scope of the invention in any way.

### [Example]

In the following Examples, all animal experiment protocols were approved by the institutional committee for animal care and related matters at Jichi Medical University, and the care of the animals was performed in accordance with the guidelines of the committee. Mice were purchased from Japan SLC. Factor VIII-deficient mice were obtained from Jackson Laboratory (USA).

### Example 1: Preparation of nucleic acids encoding B-domain-deleted human factor VIII (hFVIIISQ) and variants thereof, and coagulation factor activity of each variant hFVIIISQ

The DNA sequence of human coagulation factor VIII from which the B domain had been almost entirely removed (hFVIIISQ) was designed based on the NCBI reference sequence (NM_000132.4), and was codon-optimized and gene-synthesized by GeneArt (Thermo Scientific) (SEQ ID NO: 4).

### Ver. 1-Ver. 12

Based on the sequences of hFVIIISQ, canine FVIIISQ, porcine FVIIISQ, ovine FVIIISQ, and bovine FVIIISQ, variant Ver. 1, which was modified by gene synthesis and mutagenesis PCR so that the amino acids at 72 positions were substituted with canine-type amino acids (provided that codon optimization for humans was performed), was prepared. Furthermore, variants Ver. 2 and Ver. 3, in which the numbers of substitutions were reduced to 58 and 50 positions, respectively, were prepared. Ver. 4 having 36 substitutions, which had only the amino acid substitutions common to Ver. 2 and Ver. 3, was also prepared. In addition, Ver. 5 having 56 substitutions, obtained by removing the substitutions overlapping with the previously reported X5 and X10 mutations (WO 2014/209942) from Ver. 2; Ver. 6 having 35 substitutions, obtained by removing the substitution overlapping with the X5 mutation from Ver. 4; Ver. 7 having 51 substitutions, obtained by removing the substitutions overlapping with the previously reported JF12 mutations (Mol Ther Methods Clin Dev. 2020 Jan 15; 17:328-336) from Ver. 2; Ver. 8 having 32 substitutions, obtained by removing the substitutions overlapping with the JF12 mutations from Ver. 4; Ver. 9 having 49 substitutions, obtained by removing the substitutions overlapping with the X5, X10, and JF12 mutations from Ver. 2; Ver. 10 having 31 substitutions, obtained by removing the substitutions overlapping with the X5 and JF12 mutations from Ver. 4; Ver. 11 having 28 substitutions, obtained by reducing the number of substitutions by three positions from Ver. 10; and Ver. 12 having 13 substitutions, obtained by reducing the number of substitutions by 15 positions from Ver. 11, were prepared (the amino acid substitutions of Ver. 1 to Ver. 12 are summarized in Fig. 1). For comparison, a variant having the previously reported X5 mutations (provided that codon optimization for humans was performed) was also prepared.

After cloning, each hFVIIISQ was introduced into a plasmid (p1.1HCRhAAT) carrying an HCRhAAT promoter in which HCR, a liver-specific enhancer of the ApoE gene, was linked to the human α1-antitrypsin promoter.

For evaluation of FVIII expression by introduction of each FVIII gene (Ver. 1 to Ver. 10), FVIII transiently secreted into the culture supernatant was measured by plasmid transfection into Huh7 cells (a human liver cancer cell line). As the medium for the Huh7 cells, RPMI-1640 (Sigma) supplemented with 10% FBS, GlutaMAX (Thermo Scientific), and penicillin-streptomycin was used. Huh7 cells at 1.5 × 10^5 cells/well were seeded in a 24-well plate, and transfection using Lipofectamine 3000 (Thermo Scientific) was performed on the following day. The medium was replaced on the day following the transfection, and, on the following day, the medium after 24 hr of culture was collected. The collected medium was centrifuged, and the supernatant thereof was used as a sample for measurement of factor VIII activity and antigen.

The activity of factor VIII was measured by a synthetic substrate method using a fully automated blood coagulation analyzer CS-1600 (Sysmex).

AAV8 vectors carrying the respective FVIII genes (Ver. 1, 2, 4, and 10) were administered to factor VIII-deficient mice at a vector dose of 1×10⁸ vg per 1 g of mouse body weight. Factor VIII activity in the mouse plasma was measured by a synthetic substrate method.

The results are shown in Fig. 2. The coagulation factor activity was increased in all variants as compared with wild-type hFVIIISQ. In particular, Ver. 4 exhibited high activity in both the in vitro and in vivo experiments.

### Example 2: Identification of mutation sites important for the increase in activity of Ver. 4

The number of substitutions in Ver. 4 was progressively reduced, and variant S12 having 12 substitutions was identified based on a one-stage clotting assay. Furthermore, the number of substitutions was reduced, and PNPL (L152P, K213N, S367P, and F2196L) was identified based on a synthetic substrate assay. S7 having seven substitutions, obtained by removing five positions overlapping with the X5 and JF12 mutations from S12; S5 and S3, in which the numbers of substitutions were further reduced to five and three, respectively; and S8, S6, and S4, obtained by adding the L152P substitution to S7, S5, and S3, respectively, were prepared. Plasmid transfection was performed in the same manner as in Example 1, and the coagulation factor activity in the culture supernatant was measured by a one-stage clotting assay and a synthetic substrate assay. These results are shown in Fig. 3. S12, S8, and S6 containing the PNPL mutations exhibited high activity. Incidentally, Ver. 11, in which the number of substitutions in Ver. 10 was reduced to 28, also exhibited coagulation factor activity equivalent to that of Ver. 4.

Then, the effect of each amino acid substitution contained in S8 alone was similarly evaluated by a one-stage clotting assay and a synthetic substrate assay. The results are shown in Fig. 4. L152P, K213N, S367P, S727P, or F2196L increased the coagulation factor activity as compared with the wild type.

### Example 3: Substitution of PNPL variants with other amino acids

A series of variants (XNPL, PXPL, PNXL, and PNPX) in which any one of the substitution sites of PNPL was substituted with each of the other 19 amino acids was prepared, plasmid transfection was performed in the same manner as in Example 1, and the coagulation factor activity in the culture supernatant was measured by a one-stage clotting assay and a synthetic substrate assay. These results are shown in Figs. 5-1 to 5-4. It was revealed that substitution of L152 with Q, V, M, or the like, K213 with N, H, or the like, S367 with N, Q, or the like, or F2196 with M or the like also exhibited high activity similar to that of PNPL.

### Example 4: Administration experiment in FVIII KO mice

The Ver. 4, 10, and 11, S12, S8, and PNPL genes, which exhibited particularly high activity in Examples 1 and 2, were carried on AAV8 vectors and administered to FVIII-knockout hemophilia A model mice. Blood was collected four weeks later, and the coagulation factor activity was measured by a one-stage clotting assay and a synthetic substrate assay. In addition, the amount of antigen was measured using ELISA. The results are shown in Fig. 6. S12 and PNPL having the PNPL mutations substantially retained the activity value of Ver. 4, but the amounts of antigen were markedly smaller. Therefore, it was suggested that these variants had increased specific activity.

### Example 5: Localization of FVIII inside and outside FVIII-expressing cells

Ver. 4, S12, and PNPL were transfected into Huh7 cells, and the amounts of FVIII present in the culture supernatant and cell extract were analyzed by Western blotting. The results are shown in Fig. 7-1. In wild-type hFVIIISQ, intracellular retention of the light chain was observed, whereas in Ver. 4, the retention had disappeared. In S12 and PNPL, the intracellular retention of the light chain was not improved. The present inventors predicted that the improvement in the intracellular retention of the light chain in Ver. 4 might be due to the mutations at two positions, I1668F and D1681G, prepared an FVIIISQ variant containing only these two substitutions, and similarly examined the amount of FVIII present in the cell extract. As a result, as shown in Fig. 7-2, it was demonstrated that the FG mutations improved the intracellular retention of the light chain.

### Example 6: Identification of mutation sites important for the high secretion and high activity of Ver. 4 (2)

Based on Ver. 11, Ver. 12 (Fig. 1), in which the number of substitutions was reduced to 13 based on the synthetic substrate assay, was identified. The results are shown in Fig. 8.

Based on Ver. 12, the number of substitutions was further reduced, and highly secreting variants having 8 to 15 substitutions (F8-Tochigi-3 (NPL), 5, 8, 10, 11, 13, and 15) were prepared (the amino acid substitutions of these variants are shown in Fig. 9). Plasmid transfection was performed in the same manner as in Example 1, and the coagulation factor activity in the culture supernatant was measured by a one-stage clotting assay and a synthetic substrate assay. In addition, the amount of antigen in the culture supernatant was measured using ELISA. The results are shown in Fig. 10. In the Figure, F8-Tochigi-28 indicates the same variant as Ver. 11.

### Example 7: Construction of a high-expression-type hFVIII gene

The nucleotide sequences of codon-optimized wild-type hFVIII DNA (SEQ ID NO: 4) and Ver. 4 variant DNA (SEQ ID NO: 2) contain a large number of CpG sequences within the sequences. The present inventors predicted that these CpG sequences might induce an immune response in a host and impair the expression of FVIII, and designed nucleotide sequences from which all of the CpG sequences had been removed without changing the amino acid sequences of FVIII encoded thereby and which had then been codon-optimized again in consideration of other parameters such as GC content, using GeneArt Codon Optimizer (Thermo Scientific), and gene-synthesized the nucleotides. The nucleotide sequences of the CpG-removed and re-optimized wild-type hFVIII DNA and Ver. 4 variant DNA are shown in SEQ ID NOs: 5 and 3, respectively. These DNAs were linked downstream of a mouse transthyretin (mTTR) promoter sequence (SEQ ID NO: 7), from which CpG sequences had also been removed, carried on AAV6 vectors, subjected to plasmid transfection in the same manner as in Example 1, and introduced into Huh7 cells. In addition, the expression cassettes were carried on AAV8 vectors and administered to mice. The coagulation factor activity in the culture supernatant and blood was measured by a one-stage clotting assay and a synthetic substrate assay. The results are shown in FIGS. 11-1 and 11-2. For both Ver. 4 and the wild type, the expression efficiency was markedly increased by removing the CpG sequences.

### Example 8: Administration to a cynomolgus monkey model

The DNA prepared in Example 7, in which the CpG-removed and re-optimized Ver. 4 variant coding sequence was linked downstream of the mTTR promoter sequence from which CpG sequences had been removed, was inserted into an AAV8 vector and administered, at the vector dose (2×10¹² vg/kg) used in a clinical trial (NCT0300183) conducted by University College London, to cynomolgus monkey hemophilia models to which immunosuppressive agents (prednisolone at 1 mg/kg/day and tacrolimus at 0.05 mg/kg/day) had been administered, and factor VIII activity and the amount of antigen were measured at intervals of one or two weeks after administration. The results are shown in Fig. 12-1. Although there were differences in the course among the individual animals, factor VIII activity was markedly increased after vector administration in all of the individual animals.

In addition, the above DNA was inserted into an AAV5 vector and administered, at vector doses of one-tenth (6×10¹² vg/kg) and one-thirtieth (2×10¹² vg/kg) of Roctavian^{®} (a therapeutic agent for hemophilia A marketed by BioMarin), to similarly treated cynomolgus monkey models, and factor VIII activity and the amount of antigen were measured at intervals of one or two weeks after administration. The results are shown in Fig. 12-2. At both doses, factor VIII activity was markedly increased after vector administration.

### Example 9: Sequence optimization of expression cassettes

An AAV6 vector into which the DNA prepared in Example 7, in which the CpG-removed and re-optimized Ver. 4 variant coding sequence was linked downstream of the mTTR promoter sequence from which CpG sequences had been removed, had been inserted, the vector further containing an SV40 polyadenylation signal downstream of the coding sequence and containing, as an expression cassette, the nucleotide sequence shown in SEQ ID NO: 8 (original); an AAV6 vector containing, as an expression cassette, a nucleotide sequence (SEQ ID NO: 9) in which the sequence between the promoter and the coding sequence was truncated (v1mTTRp); and AAV6 vectors containing, as expression cassettes, three nucleotide sequences (SEQ ID NOs: 10, 11, and 12) in which the 3'-terminal side of the polyadenylation signal was truncated to different lengths (v1-v1, v1-v2, and v1-v3, respectively), were introduced into Huh7 cells. The medium was replaced after 24 hr, and after a further 24 hr, the culture supernatant and the cells were separately collected, and the factor VIII activity and the amount of antigen in the supernatant and the amount of AAV genome per cell were measured. The results are shown in Fig. 13. The activity was increased by truncating the sequence between the promoter and the coding sequence, and the activity was further improved by combining the truncating of the polyadenylation signal. The activity was highest in v1-v2. However, when the 3' end of the polyadenylation signal was deleted to the extent of v1-v3, factor VIII activity was greatly decreased.

### [Industrial Applicability]

The variant of the present invention, a nucleic acid encoding the same, an expression vector containing the nucleic acid, and a liver cell into which the expression vector has been introduced are useful for in vivo or ex vivo gene therapy for hemophilia A, mRNA medicaments, and the like. In addition, a recombinant human factor VIII variant obtained by culturing a host cell into which the expression vector has been introduced is useful as a biological product for treating hemophilia A. Furthermore, a high-expression-type human factor VIII-encoding nucleic acid is useful for gene therapy for hemophilia A and mRNA medicaments, and is also useful for producing a host that highly produces recombinant human factor VIII.

## Claims

1. A human factor VIII variant in which at least one amino acid residue selected from the group consisting of K213, S367, and F2196 of wild-type human factor VIII is substituted with other amino acid, and which has high specific activity and/or secretory expression efficiency as compared with the wild-type.

2. The variant according to claim 1, wherein the amino acid substitution at K213 is K213N or K213H, and/or
the amino acid substitution at S367 is S367P, S367N or S367Q, and/or
the amino acid substitution at F2196 is F2196L or F2196M.

3. The variant according to claim 2, comprising amino acid substitutions of K213N, S367P, and F2196L.

4. The variant according to any one of claims 1 to 3,
wherein at least one amino acid residue selected from the group consisting of R-5, P25, A28, L152, M217, W228, Q410, Y487, R489, F501, M539, I566, L603, I642, S727, A736, S1657, Q1659, E1661, I1668, D1681, R1776, H1859, A1993, H2007, N2019, K2085, K2207, F2275, S2296, V2314, Q2316 and M2321 is further substituted with other amino acid.

5. The variant according to claim 4, comprising at least one amino acid substitution selected from the group consisting of R-5P, P25H, A28T, L152A, L152D, L152E, L152H, L152I, L152M, L152N, L152P, L152Q or L152V, M217T, W228Q, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, K2207Q, F2275L, S2296A, V2314A, Q2316H, and M2321L.

6. The variant according to claim 3, further comprising amino acid substitution of L152A, L152D, L152E, L152H, L152I, L152M, L152N, L152P, L152Q, or L152V, and/or
I1668F and D1681G.

7. The variant according to claim 1, wherein the amino acid substitution at K213 is K213N,
the variant further comprises amino acid substitution of M539L, and optionally at least one amino acid substitution within the A2 domain selected from the group consisting of I566M, L603P, and I642V, and
further comprises amino acid substitutions of K2207Q, Q2316H, and M2321L, and optionally at least one amino acid substitution within the C2 domain selected from the group consisting of F2275L, S2296A, and V2314A.

8. The variant according to claim 7, further comprising amino acid substitutions of I1668F and D1681G.

9. The variant according to claim 3, further comprising amino acid substitutions of L152P, Y487H, and L603P, and optionally at least one amino acid substitution selected from the group consisting of S727P, Q1659E, H1859R, A1993V, H2007Q, N2019K, and K2085M.

10. The variant according to claim 3, further comprising amino acid substitutions of M539L, I566M, L603P, I642V, K2207Q, F2275L, S2296A, V2314A, Q2316H, and M2321L.

11. The variant according to claim 10, further comprising amino acid substitutions of I1668F and D1681G.

12. A human factor VIII variant having the following amino acid substitutions (a) to (n) in wild-type human factor VIII:
(a) K213N, S367P and F2196L
(b) K213N, S367P, F2196L, I1668F and D1681G
(c) K213N, M539L, I566M, I642V, K2207Q, F2275L, Q2316H and M2321L
(d) K213N, M539L, I566M, L603P, I642V, F2196L, K2207Q, F2275L, Q2316H and M2321L
(e) K213N, M539L, I566M, I642V, F2196L, K2207Q, F2275L, S2296A, V2314A, Q2316H and M2321L
(f) K213N, M539L, I566M, I642V, I1668F, D1681G, F2196L, K2207Q, F2275L, S2296A, V2314A, Q2316H and M2321L
(g) L152P, K213N, S367P and F2196L
(h) L152P, K213N, S367P, Y487H, L603P and F2196L
(i) L152P, K213N, S367P, Y487H, L603P, S727P, Q1659E and F2196L
(j) L152P, K213N, S367P, Y487H, L603P, S727P, Q1659E, H1859R, A1993V, H2007Q, K2085M and F2196L
(k) K213N, S367P, M539L, I566M, L603P, I642V, F2196L, K2207Q, F2275L, S2296A, V2314A, Q2316H and M2321L
(l) P25H, A28T, K213N, M217T, S367P, Q410L, Y487H, F501M, M539L, I566M, L603P, I642V, S727P, A736V, S1657P, Q1659E, E1661K, I1668F, D1681G, R1776K, N2019K, F2196L, K2207Q, F2275L, S2296A, V2314A, Q2316H and M2321L
(m) R-5P, P25H, A28T, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, N2019K, F2196L, K2207Q, F2275L, S2296A, V2314A, Q2316H and M2321L
(n) R-5P, P25H, A28T, L152P, K213N, M217T, W228Q, S367P, Q410L, Y487H, R489G, F501M, M539L, I566M, L603P, I642V, S727P, A736V, S1657P, Q1659E, E1661K, I1668F, D1681G, R1776K, H1859R, A1993V, H2007Q, N2019K, K2085M, F2196L, K2207Q, F2275L, S2296A, V2314A, Q2316H and M2321L.

13. The variant according to any one of claims 1 to 12,
wherein the B domain is deleted.

14. The variant according to claim 13, wherein the deletion of the B domain is a deletion of the amino acid sequence from position 763 to position 1656 in the amino acid sequence represented by SEQ ID NO: 1.

15. A nucleic acid encoding the variant according to any one of claims 1 to 14.

16. The nucleic acid according to claim 15, which is codon-optimized for humans.

17. A nucleic acid that is either the nucleic acid according to claim 16 or a nucleic acid encoding wild-type human factor VIII and codon-optimized for humans, and that has been further modified so as not to contain CpG sequences without changing the amino acid sequence encoded thereby.

18. An expression vector comprising the nucleic acid according to any one of claims 15 to 17.

19. The expression vector according to claim 18, wherein the nucleic acid is under the control of a liver-specific promoter.

20. The expression vector according to claim 18 or 19,
wherein the promoter has been modified so as not to contain CpG sequences.

21. The expression vector according to any one of claims 18 to 20, wherein the vector is a viral vector.

22. The expression vector according to claim 21, wherein the virus is a liver-tropic adeno-associated virus.

23. A host cell into which the expression vector according to any one of claims 18 to 22 has been introduced.

24. A method for producing human factor VIII or a variant thereof, comprising culturing the host cell according to claim 23 and recovering the human factor VIII or a variant thereof from the obtained culture.

25. A medicament comprising the variant according to any one of claims 1 to 14, the nucleic acid according to any one of claims 15 to 17, the expression vector according to any one of claims 18 to 22, or the host cell according to claim 23.

26. The medicament according to claim 25, for the treatment of hemophilia A.
